# EUROPEAN PATENT SPECIFICATION

(11) **EP 2 244 706 B1**
(45) Date of publication and mention of the grant of the patent: **06.04.2016**
(21) Application number: 09715751.5
(22) Date of filing: 02.02.2009
(51) Int. Cl.: C07D 409/14, A61K 31/4155, A61K 31/4178, A61K 31/4184, A61K 31/4196, A61K 31/427, A61K 31/433, A61K 31/4439, A61K 31/4436, A61K 31/501, A61K 31/506, A61P 3/10, C07D 417/12, C07D 409/12, C07D 413/12

(54) **GLUCOKINASE ACTIVATORS**
GLUCOKINASE-AKTIVATOREN
ACTIVATEURS DE LA GLUCOKINASE

(30) Priority: 25.02.2008 EP 08003356
(43) Date of publication of application: 03.11.2010
(73) Proprietor: Merck Patent GmbH, 64271 Darmstadt (DE)
(72) Inventor: BURGDORF, Lars Thore, 60389 Frankfurt am Main (DE); EMDE, Ulrich, 64291 Darmstadt (DE); GLEITZ, Johannes, 64289 Darmstadt (DE); BEIER, Norbert, 64354 Reinheim (DE); CHARON, Christine, F-91940 Gometz-le-Chatel (FR)
(86) International application number: PCT/EP2009/000653
(87) International publication number: WO 2009/106203

(56) References cited:
- EP-A- 1 873 144
- WO-A-03/041708
- WO-A-2006/134317
- WO-A-2007/022258
- WO-A-2007/130075
- WO-A-2008/120744
- US-A1- 2004 054 186
- DATABASE REGISTRY Chemical Library Supplier: Enamine 30 November 2004 (2004-11-30), XP002521523 retrieved from STN accession no. 790725-27-8
- DATABASE REGISTRY Chemical Library Supplier: Aurora Fine Chemicals 27 April 2007 (2007-04-27), XP002521524 retrieved from STN accession no. 932902-06-2
- WAN ET AL: "Probing acid replacements of thiophene PTP1B inhibitors" BIOORGANIC & MEDICINAL CHEMISTRY LETTERS, PERGAMON, ELSEVIER SCIENCE, GB, vol. 17, no. 10, 27 April 2007 (2007-04-27), pages 2913-2920, XP022049613 ISSN: 0960-894X

## Description

### BACKGROUND OF THE INVENTION

The invention had the object of finding novel compounds having valuable properties, in particular those which can be used for the preparation of medicaments.

The present disclosure relates to compounds that are useful in the treatment and/or prevention of diseases mediated by deficient levels of glucokinase activity, such as diabetes mellitus, and methods of preparing such compounds. Also disclosed are methods of treating diseases and disorders characterized by underactivation of glucokinase activity or which can be treated by activating glucokinase, comprising administering an effective amount of a compound of this invention.

The identification of small compounds which specifically activate, regulate and/or modulate signal transduction of glucokinase is therefore desirable and an aim of the present invention. Moreover, aim of this invention was the preparation of new compounds for the prevention and/or treatment of Diabetes Type 1 and 2, obesity, neuropathy and/or nephropathy.

Surprisingly we have found that certain thiophene derivatives activate glucokinase; therefore, these compounds are especially suitable for the prevention and treatment of Diabetes Type 1 and 2, obesity, neuropathy and/or nephropathy. It has been found that the compounds according to the invention and salts thereof have very valuable pharmacological properties while being well tolerated.

In particular, they exhibit glucokinase activating effects.

The present invention therefore relates to compounds according to the invention as medicaments and/or medicament active ingredients in the treatment and/or prophylaxis of the said diseases and to the use of compounds according to the invention for the preparation of a pharmaceutical for the treatment and/or prophylaxis of the said diseases.

The host or patient may belong to any mammal species, for example a primate species, particularly humans; rodents, including mice, rats and hamsters; rabbits; horses, cows, dogs, cats, etc. Animal models are of interest for experimental investigations, where they provide a model for the treatment of a human disease.

Diabetes mellitus (DM) is a progressive disease often associated with obesity characterized by insulin deficiency and insulin resistance or both. The fasting and post-prandial blood glucose is elevated, exposing the patient to acute and chronic complications (micro- and macro-vascular) leading to blindness, kidney failure, heart disease, stroke and amputations. Improving glycemic control has been demonstrated to lower the risk of these complications. Owing to the progressive nature of the disease, an evolving treatment strategy is necessary to maintain glycemic control. There are two forms of diabetes mellitus: type 1, or juvenile diabetes or insulin-dependent diabetes mellitus (IDDM), and type 2, or adult-onset diabetes or non insulin-dependent diabetes mellitus (NIDDM). Type 1 diabetes patients have an absolute insulin insufficiency due to the immunological destruction of pancreatic p cells that synthesize and secrete insulin. Type 2 diabetes is more complex in etiology and is characterized by a relative insulin deficiency, reduced insulin action, and insulin resistance. Early-onset NIDDM or maturity-onset diabetes of the young (MODY) shares many features of the most common form of NIDDM whose onset occurs in the midlife (Rotter et al 1990). A clear mode of inheritance (autosomal dominant) has been observed for MODY. At least, 3 distinct mutations have been identified in MODY families (Bell et al. 1996).

The importance of Glucokinase (GK) in glucose homeostasis has been demonstrated by the association of GK mutants with diabetes mellitus in humans (MODY-2) and by alteration in glucose metabolism in transgenic mice and gene knock-out mice (Froguel et al. 2003; Bali et al. 1995, Postic et al. 1999).

GK, also known as hexokinase IV or D, is one of four hexokinase isozymes that metabolize glucose to glucose 6-phosphate [Wilson, 2004]. GK is known to be expressed in neural/neuroendocrine cells, hepatocytes and pancreatic cells and plays a central role in whole body homeostasis [Matschinsky et al. 1996; 2004]. GK plays an important role as a glucose sensor for controlling plasma glucose homeostasis by enhancing insulin secretion from pancreatic β-cells and glucose metabolism in the liver but also by increasing GLP1 secretion from L-Cells. β-cells, glucose-sensing in the arcuate (ARC) hypothalamic nucleus may depend on GK to detect a rise in glucose and facilitate glucose-induced-insulin secretion.

The multiple mechanism of action suggests that GK activators will exert their biological effects in diabetic and obese patients by improving the overall body glucose awareness which provides rational expectations that enhancement of GK activity would be a novel therapeutic strategy for metabolic disorders. It is anticipated that GK activators will restore appropriated pancreatic hormones and incretin secretion coupled with a suppression of hepatic glucose production without inducing severe hypoglycemia.

### Bibliography

Wilson JE: The hexokinase gene family. In Glucokinase and Glycemic
Disease: From Basics to Novel Therapeutics. Front Diabetes. Vol. 16.
Matschinsky FM, Magnuson MA, Eds. Basel, Karger, 2004
Matschinsky, F. M. Diabetes 1996, 45, 223-41.
Matschinsky F.M.; Magnuson M.A. eds. Glucokinase and Glycemic
Disease: From Basics to Novel Therapeutics. Basel:Karger, 2004
Rotter et al. Diabetes mellitus (1990): Theory and practice Rifkin and Porte (Eds) NY, 378-413
Bell et al 1996
Froguel et al. 2003
Bali et al. 1995
Postic et al. 1999

The following structures are known in the art. They have however never been described as GK activators.
5-Propyl-thiophene-3-carboxylic acid (5-chloro-pyridin-2-yl)-amide,
5-Propyl-thiophene-3-carboxylic acid thiazol-2-ylamide,
Thiophene-3-carboxylic acid thiazol-2-ylamide,
Thiophene-2-carboxylic acid thiazol-2-ylamide,
6-Methyl-4,5,6,7-tetrahydro-benzo[b]thiophene-3-carboxylic acid thiazol-2-ylamide,
4-Methyl-2-[(5-propyl-thiophene-3-carbonyl)-amino]-thiazole-5-carboxylic acid ethyl ester,
5-Methyl-thiophene-2-carboxylic acid thiazol-2-ylamide,
5-Propyl-thiophene-3-carboxylic acid isoxazol-3-ylamide, and
4-Dimethylsulfamoyl-5-methyl-thiophene-2-carboxylic acid thiazol-2-ylamide.

### SUMMARY OF THE INVENTION

The compounds according to the invention and also the starting materials for their preparation are, in addition, prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants known per se, which are not mentioned here in greater detail.

If desired, the starting materials can also be formed in situ so that they are not isolated from the reaction mixture, but instead are immediately converted further into the compounds according to the invention.

The starting compounds are generally known. If they are novel, however, they can be prepared by methods known per se.

Compounds of the invention can for example be obtained by:
Route A 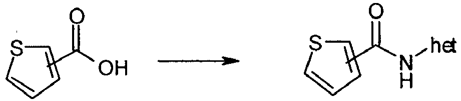
Route B 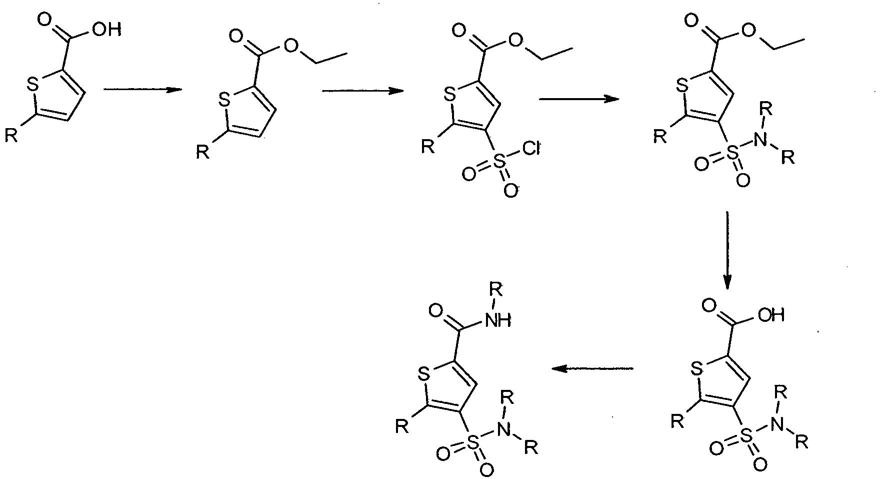
Route C

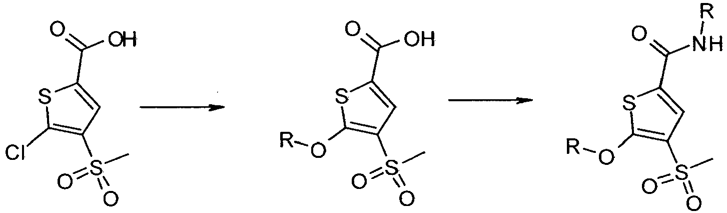

These reactions are carried out by methods which are known to the person skilled in the art.

The reaction is generally carried out in an inert solvent, in the presence of an acid-binding agent, preferably an alkali or alkaline-earth metal hydroxide, carbonate or bicarbonate or another salt of a weak acid of the alkali or alkaline-earth metals, preferably of potassium, sodium, calcium or caesium. The addition of an organic base, such as triethylamine, dimethylaniline, pyridine or quinoline may also be favourable.

Radicals of this type for activation of the carboxyl group in typical acylation reactions are described in the literature (for example in the standard works, such as Houben-Weyl, Methoden der organischen Chemie [Methods of Organic Chemistry], Georg-Thieme-Verlag, Stuttgart).

Activated esters are advantageously formed in situ, for example through addition of HOBt or N-hydroxysuccinimide.

Suitable inert solvents are, for example, hydrocarbons, such as hexane, petroleum ether, benzene, toluene or xylene; chlorinated hydrocarbons, such as trichforoethylene, 1,2-dichloroethane, carbon tetrachloride, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether, ethylene glycol dimethyl ether (diglyme); ketones, such as acetone or butanone; amides, such as acetamide, dimethylacetamide or dimethylformamide (DMF); nitriles, such as acetonitrile; sulfoxides, such as dimethyl sulfoxide (DMSO); carbon disulfide; carboxylic acids, such as formic acid or acetic acid; nitro compounds, such as nitromethane or nitrobenzene; esters, such as ethyl acetate, or mixtures of the said solvents.

Depending on the conditions used, the reaction time is between a few minutes and 14 days, the reaction temperature is between about -30° and 140°, normally between -10° and 110°, in particular between about 20° and about 100°.

Other radicals can be converted by reducing nitro groups (for example by hydrogenation on Raney nickel or Pd/carbon in an inert solvent, such as methanol or ethanol) to amino groups or hydrolysing cyano groups to COOH groups.

Furthermore, free amino groups can be acylated in a conventional manner using an acid chloride or anhydride or alkylated using an unsubstituted or substituted alkyl halide, advantageously in an inert solvent, such as dichloromethane or THF, and/or in the presence of a base, such as triethylamine or pyridine, at temperatures between -60 and +30°C.

Ester groups can be saponified, for example, using NaOH or KOH in water, water/THF or water/dioxane at temperatures between 0 and 100°C. Carboxylic acids can be converted, for example using thionyl chloride, into the corresponding carboxylic acid chlorides, and the latter can be converted into carboxamides. Elimination of water therefrom in a known manner gives carbonitriles.

### Pharmaceutical salts and other forms

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. Pharmaceutically acceptable salt forms of the compounds of the invention are for the most part prepared by conventional methods. If the compound contains a carboxyl group, one of its suitable salts can be formed by reacting the compound with a suitable base to give the corresponding base-addition salt. Such bases are, for example, alkali metal hydroxides, including potassium hydroxide, sodium hydroxide and lithium hydroxide; alkaline earth metal hydroxides, such as barium hydroxide and calcium hydroxide; alkali metal alkoxides, for example potassium ethoxide and sodium propoxide; and various organic bases, such as piperidine, diethanolamine and N-methyl-glutamine. The aluminium salts of the compounds of the invention are likewise included. In the case of certain compounds of the invention, acid-addition salts can be formed by treating these compounds with pharmaceutically acceptable organic and inorganic acids, for example hydrogen halides, such as hydrogen chloride, hydrogen bromide or hydrogen iodide, other mineral acids and corresponding salts thereof, such as sulfate, nitrate or phosphate and the like, and alkyl- and monoarylsulfonates, such as ethanesulfonate, toluenesulfonate and benzenesulfonate, and other organic acids and corresponding salts thereof, such as acetate, trifluoroacetate, tartrate, maleate, succinate, citrate, benzoate, salicylate, ascorbate and the like. Accordingly, pharmaceutically acceptable acid-addition salts of the compounds of the invention include the following: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, palmoate, pectinate, persulfate, phenylacetate, 3-phenylpropionate, phosphate, phosphonate, phthalate, but this does not represent a restriction. Furthermore, the base salts of the compounds according to the invention include aluminium, ammonium, calcium, copper, iron(III), iron(II), lithium, magnesium, manganese(III), manganese(II), potassium, sodium and zinc salts, but this is not intended to represent a restriction. Of the above-mentioned salts, preference is given to ammonium; the alkali metal salts sodium and potassium, and the alkaline earth metal salts calcium and magnesium. Salts of the compounds of the invention which are derived from pharmaceutically acceptable organic non-toxic bases include salts of primary, secondary and tertiary amines, substituted amines, also including naturally occurring substituted amines, cyclic amines, and basic ion exchanger resins, for example arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris-(hydroxymethyl)methylamine (tromethamine), but this is not intended to represent a restriction.

Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

The above-mentioned pharmaceutical salts which are preferred include acetate, trifluoroacetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate and tromethamine, but this is not intended to represent a restriction.

The acid-addition salts of basic compounds of the invention are prepared by bringing the free base form into contact with a sufficient amount of the desired acid, causing the formation of the salt in a conventional manner. The free base can be regenerated by bringing the salt form into contact with a base and isolating the free base in a conventional manner. The free base forms differ in a certain respect from the corresponding salt forms thereof with respect to certain physical properties, such as solubility in polar solvents; for the purposes of the invention, however, the salts otherwise correspond to the respective free base forms thereof.

As mentioned, the pharmaceutically acceptable base-addition salts of the compounds of the invention are formed with metals or amines, such as alkali metals and alkaline earth metals or organic amines. Preferred metals are sodium, potassium, magnesium and calcium. Preferred organic amines are N,N'-dibenzylethylenediamine, chloroprocaine, choline, diethanolamine, ethylenediamine, N-methyl-D-glucamine and procaine. The base-addition salts of acidic compounds according to the invention are prepared by bringing the free acid form into contact with a sufficient amount of the desired base, causing the formation of the salt in a conventional manner. The free acid can be regenerated by bringing the salt form into contact with an acid and isolating the free acid in a conventional manner. The free acid forms differ in a certain respect from the corresponding salt forms thereof with respect to certain physical properties, such as solubility in polar solvents; for the purposes of the invention, however, the salts otherwise correspond to the respective free acid forms thereof.

If a compound according to the invention contains more than one group which is capable of forming pharmaceutically acceptable salts of this type, the invention also encompasses multiple salts. Typical multiple salt forms include, for example, bitartrate, diacetate, difumarate, dimeglumine, diphosphate, disodium and trihydrochloride, but this is not intended to represent a restriction.

With regard to that stated above, it can be seen that the expression "pharmaceutically acceptable salt" in the present connection is taken to mean an active ingredient which comprises a compound of the invention in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

Compounds according to the invention may be chiral owing to their molecular structure and may accordingly occur in various enantiomeric forms. They can therefore exist in racemic or in optically active form. Since the pharmaceutical activity of the racemates or stereoisomers of the compounds according to the invention may differ, it may be desirable to use the enantiomers. In these cases, the end product or even the intermediates can be separated into enantiomeric compounds by chemical or physical measures known to the person skilled in the art or even employed as such in the synthesis.

In the case of racemic amines, diastereomers are formed from the mixture by reaction with an optically active resolving agent. Examples of suitable resolving agents are optically active acids, such as the R and S forms of tartaric acid, diacetyltartaric acid, dibenzoyltartaric acid, mandelic acid, malic acid, lactic acid, suitably N-protected amino acids (for example N-benzoylproline or N-benzenesulfonylproline), or the various optically active camphorsulfonic acids. Also advantageous is chromatographic enantiomer resolution with the aid of an optically active resolving agent (for example dinitrobenzoylphenylglycine, cellulose triacetate or other derivatives of carbohydrates or chirally derivatised methacrylate polymers immobilised on silica gel). Suitable eluents for this purpose are aqueous or alcoholic solvent mixtures, such as, for example, hexane/isopropanol/ acetonitrile, for example in the ratio 82:15:3.

The invention furthermore relates to the use of the compounds and/or physiologically acceptable salts thereof for the preparation of a medicament (pharmaceutical composition), in particular by non-chemical methods. They can be converted into a suitable dosage form here together with at least one solid, liquid and/or semi-liquid excipient or adjuvant and, if desired, in combination with one or more further active ingredients.

The invention furthermore relates to medicaments comprising at least one compound according to the invention and/or salts, solvates and stereoisomers thereof, including mixtures thereof in all ratios, and optionally excipients and/or adjuvants.

Pharmaceutical formulations can be administered in the form of dosage units which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the disease condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water and the like. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting moulds. The lubricated mixture is then pressed to give tablets. The compounds according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a prespecified amount of the compounds. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The compounds according to the invention and salts or solvates thereof can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds according to the invention and salts or solvates thereof can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropylmethacrylamidophenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis, as described in general terms in Pharmaceutical Research, 3(6), 318 (1986).

Pharmaceutical compounds adapted for topical administration can be formulated as ointments, creams, suspensions, lotions, powders, solutions, pastes, gels, sprays, aerosols or oils.

For the treatment of the eye or other external tissue, for example mouth and skin, the formulations are preferably applied as topical ointment or cream. In the case of formulation to give an ointment, the active ingredient can be employed either with a paraffinic or a water-miscible cream base. Alternatively, the active ingredient can be formulated to give a cream with an oil-in-water cream base or a water-in-oil base.

Pharmaceutical formulations adapted for topical application to the eye include eye drops, in which the active ingredient is dissolved or suspended in a suitable carrier, in particular an aqueous solvent.

Pharmaceutical formulations adapted for topical application in the mouth encompass lozenges, pastilles and mouthwashes.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil.

Pharmaceutical formulations adapted for administration by inhalation encompass finely particulate dusts or mists, which can be generated by various types of pressurised dispensers with aerosols, nebulisers or insufflators.

Pharmaceutical formulations adapted for vaginal administration can be administered as pessaries, tampons, creams, gels, pastes, foams or spray formulations.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary.

Injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound of the present invention depends on a number of factors, including, for example, the age and weight of the human or animal, the precise disease condition which requires treatment, and its severity, the nature of the formulation and the method of administration, and is ultimately determined by the treating doctor or vet. However, an effective amount of a compound according to the invention is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as an individual dose per day or usually in a series of part-doses (such as, for example, two, three, four, five or six) per day, so that the total daily dose is the same. An: effective amount of a salt or solvate thereof can be determined as the fraction of the effective amount of the compound according to the invention *per se.* It can be assumed that similar doses are suitable for the treatment of other conditions mentioned above.

### USE

The present compounds are suitable as pharmaceutical active ingredients for mammals, in particular for humans, in the treatment of Diabetes Typ 1 and 2, obesity, neuropathy and/or nephropathy.

The invention thus relates to the use of compounds according to Claim 1 and to salts solvates and stereoisomers, including mixtures thereof in all ratios, for the preparation of a medicament for the treatment of Diabetes Type 1 and 2, obesity, neuropathy and/or nephropathy.

The compounds of the present invention can be used as prophylactics or therapeutic agents for treating diseases or disorders mediated by deficient levels of glucokinase activity or which can be treated by activating glucokinase including, but not limited to, diabetes mellitus, impaired glucose tolerance, IFG (impaired fasting glucose) and IFG (impaired fasting glycemia), as well as other diseases and disorders such as those discussed below.

Furthermore, the compounds of the present invention can be also used to prevent the progression of the borderline type, impaired glucose tolerance, IFG (impaired fasting glucose) or IFG (impaired fasting glycemia) to diabetes mellitus.

The compounds of the present invention can be also used as prophylactics or therapeutic agents of diabetic complications such as, but not limited to, neuropathy, nephropathy, retinopathy, cataract, macroangiopathy, osteopenia, diabetic hyperosmolar coma), infectious diseases (e.g., respiratory infection, urinary tract infection, gastrointestinal tract infection, dermal soft tissue infection, lower limb infection etc.), diabetic gangrene, xerostomia, decreased sense of hearing, cerebrovascular disease, peripheral circulatory disturbance, etc.

The compounds of the present invention can be also used as prophylactics or therapeutic agents in the treatment of diseases and disorders such as, but not limited to, obesity, metabolic syndrome (syndrome X), hyperinsulinemia, hyperinsulinemia-induced sensory disorder, dyslipoproteinemia (abnormal lipoproteins in the blood) including diabetic dyslipidemia, hyperlipidemia, hyperlipoproteinemia (excess of lipoproteins in the blood) including type I, II-a (hypercholesterolemia), II-b, III, IV (hypertriglyceridemia) and V (hypertriglyceridemia), low HDL levels, high LDL levels, atherosclerosis and its sequelae, vascular restenosis, neurodegenerative disease, depression, CNS disorders, liver steatosis, osteoporosis, hypertension, renal diseases (e.g., diabetic nephropathy, glomerular nephritis, glomeruloscierosis, nephrotic syndrome, hypertensive nephrosclerosis, terminal renal disorder etc.), myocardiac infarction, angina pectoris, and cerebrovascular disease (e.g., cerebral infarction, cerebral apoplexy).

The compounds of the present invention can be also used as prophylactics or therapeutic agents in the treatment of diseases and disorders such as, but not limited to, osteoporosis, fatty liver, hypertension, insulin resistant syndrome, inflammatory diseases (e.g., chronic rheumatoid arthritis, spondylitis deformans, osteoarthritis, lumbago, gout, postoperative or traumatic inflammation, remission of swelling, neuralgia, pharyngolaryngitis, cystitis, hepatitis (including non-alcoholic steatohepatitis), pneumonia, inflammatory colitis, ulcerative colitis), pancreatitis, visceral obesity syndrome, cachexia (e. g., carcinomatous eachexia, tuberculous cachexia, diabetic cachexia, hemopathic cachexia, endocrinopathic cachexia, infectious cachexia, cachexia induced by acquired immunodeficiency syndrome), polycystic ovary syndrome, muscular dystrophy, tumor (e.g., leukemia, breast cancer, prostate cancer, skin cancer etc.), irritable bowel syndrome, acute or chronic diarrhea, spondylitis deformans, osteoarthritis, remission of swelling, neuralgia, pharyngolaryngitis, cystitis, SIDS, and the like.

The compounds of the present invention can be used in combination with one or more additional drugs such as described below. The dose of the second drug can be appropriately selected based on a clinically employed dose. The proportion of the compound of the present invention and the second drug can be appropriately determined according to the administration subject, the administration route, the target disease, the clinical condition, the combination, and other factors. In cases where the administration subject is a human, for instance, the second drug may be used in an amount of 0.01 to 100 parts by weight per part by weight of the compound of the present invention.

The second compound of the pharmaceutical combination formulation or dosing regimen preferably has complementary activities to the compound of the present invention such that they do not adversely affect each other. Such drugs are suitably present in combination in amounts that are effective for the purpose intended. Accordingly, another aspect described herein is a composition comprising a compound of the present invention, or a solvate, metabolite, or pharmaceutically acceptable salt or prodrug thereof, in combination with a second drug, such as described herein.

The compound of the present invention and the additional pharmaceutically active agent(s) may be administered together in a unitary pharmaceutical composition or separately and, when administered separately this may occur simultaneously or sequentially in any order. Such sequential administration may be close in time or remote in time. The amounts of the compound of the present invention and the second agent(s) and the relative timings of administration will be selected in order to achieve the desired combined therapeutic effect.

The combination therapy may provide "synergy" and prove "synergistic", i.e., the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds are administered or delivered sequentially, e.g., by different injections in separate syringes. In general, during alternation therapy, an effective dosage of each active ingredient is administered sequentially, i.e., serially, whereas in combination therapy, effective dosages of two or more active ingredients are administered together.

The compounds of the present invention can be used, for example in combination with additional drug(s) such as a therapeutic agent for diabetes mellitus, and/or a therapeutic agent for diabetic complications, as defined above.

Examples of known therapeutic agents for diabetes mellitus which can be used in combination with a compound of the present invention include insulin preparations (e.g., animal insulin preparations extracted from the bovine or swine pancreas; human insulin preparations synthesized by a genetic engineering technique using Escherichia coli or a yeast), a fragment of insulin or derivatives thereof (e.g., INS-i), agents for improving insulin resistance (e.g., pioglitazone hydrochloride, troglitazone, rosiglitazone or its maleate, GI-262570, JTT-50 1, MCC-555, YM-440, KRP-297, CS-Oil, FK-614), alpha-glucosidase inhibitors (e.g., voglibose, acarbose, miglitol, emiglitate), biguanides (e.g., phenformin, metformin, buformin), insulin secretagogues [sulfonylureas (e.g., tolbutamide, glibenclamide, gliclazide, chiorpropamide, tolazamide, acetohexamide, glyclopyramide, glimepiride, glipizide, glybuzole), repaglinide, nateglinide, mitiglinide or its calcium salt hydrate, GLP-1J, dipeptidylpeptidase IV inhibitors (e.g., NVP-DPP-278, PT-100), beta-3 agonists (e.g., CL-3 16243, SR-58611-A, UL-TG-307, SB-226552, AJ-9677, BMS-I96085, AZ-40140, etc.), amylin agonists (e.g., pramlintide), phosphotyrosine phosphatase inhibitors (e.g., vanadic acid), gluconeogenesis inhibitors (e.g., glycogen phosphorylase inhibitors, glucose-6-phosphatase inhibitors, glucagon antagonists), SGLT (sodium-glucose cotransporter) inhibitors (e.g., T-1095), and the like.

Examples of known therapeutic agents for diabetic complications include aldose reductase inhibitors (e.g., tolrestat, epairestat, zenarestat, zopobestat, minairestat, fidarestat (SNK-860), CT-i 12), neurotrophic factors (e.g., NGF, NT-3, BDNF), neurotrophic factor production secretion promoters, PKC inhibitors (e.g., LY-333531), AGE inhibitors (e.g., ALT946, pimagedine, pyratoxathine, N-phenacylthiazolium bromide (ALT766), EXO-226), active oxygen scavengers (e.g., thioctic acid), and cerebral vasodilators (e.g., tiapuride, mexiletine).

The compounds of the present invention can also be used, for example in combination with antihyperlipidemic agents. Epidemiological evidence has firmly established hyperlipidemia as a primary risk factor in causing cardiovascular disease (CVD) due to atherosclerosis. In recent years, emphasis has been placed on lowering plasma cholesterol levels, and low density lipoprotein cholesterol in particular, as an essential step in prevention of CVD.

Cardiovascular disease is especially prevalent among diabetic subjects, at least in part because of the existence of multiple independent risk factors in this population. Successful treatment of hyperlipidemia in the general population, and in diabetic subjects in particular, is therefore of exceptional medical importance. Examples of antihyperlipidemic agents include statin compounds which are cholesterol synthesis inhibitors (e.g., cerivastatin, pravastatin, simvastatin, lovastatin, atorvastatin, fluvastatin, itavastatin or their salts, etc.), squalene synthase inhibitors or fibrate compounds (e.g., bezafibrate, clofibrate, simfibrate, clinofibrate) having a triglyceride lowering action and the like.

The compounds of the present invention can also be used, for example in combination with hypotensive agents. Hypertension has been associated with elevated blood insulin levels, a condition known as hyperinsulinemia. Insulin, a peptide hormone whose primary actions are to promote glucose utilization, protein synthesis and the formation and storage of neutral lipids, also acts to promote vascular cell growth and increase renal sodium retention, among other things. These latter functions can be accomplished without affecting glucose levels and are known causes of hypertension. Peripheral vasculature growth, for example, can cause constriction of peripheral capillaries, while sodium retention increases blood volume. Thus, the lowering of insulin levels in hyperinsulinemics can prevent abnormal vascular growth and renal sodium retention caused by high insulin levels and thereby alleviates hypertension. Examples of hypotensive agents include angiotensin converting enzyme inhibitors (e.g., captopril, enalapril, delapril), angiotensin II antagonists (e.g., candesartan cilexetil, losartan, eprosartan, valsantan, termisartan, irbesartan, tasosartan), calcium antagonists (e.g., manidipine, nifedipine, nicardipine, amlodipine, efonidipine), and clonidine.

The compounds of the present invention can be used in combination with antiobesity agents. The term "obesity" implies an excess of adipose tissue. Obesity is a well-known risk factor for the development of many very common diseases such as diabetes, atherosclerosis, and hypertension. To some extent appetite is controlled by discrete areas in the hypothalamus: a feeding centre in the ventrolateral nucleus of the hypothalamus (VLH) and a satiety centre in the ventromedial hypothalamus (VMH). The cerebral cortex receives positive signals from the feeding center that stimulate eating, and the satiety center modulates this process by sending inhibitory impulses to the feeding center. Several regulatory processes may influence these hypothalamic centers. The satiety center may be activated by the increases in plasma glucose and/or insulin that follow a meal. Examples of antiobesity agents include antiobesity drugs acting on the central nervous system (e.g., dexfenfluramine, fenfluramine, phentermine, sibutramine, anfepramon, dexamphetamine, mazindol, phenyipropanolamine, clobenzorex), pancreatic lipase inhibitors (e.g. orlistat), beta-3 agonists (e.g., CL-3 16243, SR-5861 1-A, UL-TG-307, SB-226552, AJ-9677, BMS-196085, AZ-40I40), anorectic peptides (e.g., leptin, CNTF (Ciliary Neurotrophic Factor) and cholecystokinin agonists (e.g. lintitript, FPL-1 5849).

### ASSAYS

### Glucokinase activation screening assay

GK activity (human or rat enzyme) is measured by a coupled enzyme assay using pyruvate kinase (PK) and lactate dehydrogenase (LDH) as coupling enzymes. GK activity is calculated from the decline in NADH monitored photometrically with a microtiter plate (MTP) reader at 340 nm.

For screening purposes, the GK assay is routinely run in a 384-MTP format, in a total volume of 33 µl/well. 10 µl of the ATP-regeneration solution (in HEPES-buffer* , pH 7.0, 6.73 U/ml pyruvate kinase, 6.8 U/ml lactate dehydrogenase) and 10 µl of the glucokinase-/glucose solution (15 µg/ml, 6.6 mM glucose in HEPES-buffer*, pH 7.0 ; the concentration of the glucose stock-solution was 660mM in Millipore H₂O) were mixed together with 3 µl of a 10 % DMSO solution (in HEPES-buffer*, pH 7.0) containing 3.3-fold the amounts of the compounds to achieve final compound concentrations in the range between 1 nM to 30 µM (sometimes 300 µM) in the assay solution (s. below). The solutions were mixed for 5 sec, and after a centrifugation at 243xg for 5 min, the solutions were preincubated for 25 min at room temperature.

The reaction was started by the addition of 10 µl of the NADH-/ATP-solution (4.29 mM NADH, 4.95 mM ATP, in HEPES-buffer*). The MTP was shaken for 5 sec., and then, the absorbance at 340 nm was monitored continuously in a MTP-reader (TECAN Spectro fluor plus) for the next 27 min (with a MTP-cycling time of 199 sec.). The final concentrations of the various components were as follows: 49.5 mM Hepes, pH 7.0, 1.49 mM PEP,1,3 mM NADH, 49.5 mM KCI, 4.96 mM MgCl₂, 1.5 mM Mg-ATP, 1.98 mM DTT, 2.04 U/ml pyruvate kinase, 2.06 U/ml lactate-dehydrogenase, 0.91 % DMSO, 0.15 µg/well glucokinase, and test compounds in the range between 1 nM and 300 µM.

The change in the optical density (ΔOD_{340 nm}) in the presence of the compound was expressed relative to the ΔOD₃₄₀ nm, ctrl of the control incubation (in the presence of 2 mM glucose and 0.91 % DMSO), taking into account the optical density of the blank sample (incubation in the absence of 2 mM glucose). For the determination of the half maximal effective concentration (EC₅₀), the %-Ctrl-values were plotted in a semi-logarithmic graph against the conc. of the compound of interest. The data points were fitted to a sigmoid curve function (f(x) = ((%-Ctrlₘₐₓ - %-Ctrlₘᵢₙ)/(1 - (EC₅₀/X**^{n(Hill)})) + %-Ctrlₘᵢₙ)) by a non-linear regression analysis.

* Hepes-buffer (50mM Hepes, , pH 7.0, 5mM MgCl₂, 50mM KCI, 1.5 mM PEP, 0.1% BSA). DTT was added to the Hepes-buffer from a 200X stock solution (in Millipore H₂O) freshly each day. The final concentration of DTT in the Hepes-buffer is 2 mM.

### Culture of pancreatic INS-1 cells

INS-1 cells were cultured in complete medium, RPMI1640 containing 1mM sodium pyruvate, 50µM 2-mercaptoethanol, 2mM glutamine, 10mM HEPES, 100IU/mL penicillin, and 100µg/mL streptomycin (CM), supplemented with 10mM glucose, and 10% (vol/vol) heat-inactivated fetal calf serum (FCS), as described by Asfari et al. (Endocrinology 130: 167-178, 1992).

### Insulin secretion assay

INS-1 cells were plated and cultured in 48-well plates. After 2 days of culture, the medium was removed and cells were cultured for 24h with a medium change to 5mM glucose, 1% FCS. The cells were then washed with Krebs-Ringer Bicarbonate HEPES buffer (KRBH; 135mM NaCl; 3,6mM KCI; 5mM NaHCO3; 0,5mM NaH2PO4; 0,5mM MgCl2; 1,5mM CaCl2 and 10mM HEPES; pH 7,4) 0,1% BSA containing 2,8mM glucose and preincubated for 30min at 37°C in the same buffer. The cells were then washed twice and incubated for 1 h in KRBH 0,1% BSA containing 2,8 or 4,2mM glucose and different concentrations of the tested molecule. Insulin concentration in the collected supernatants was measured with ELISA using rat insulin antibody (Insulin Rat Elit PLUS, cat. ref 10-1145-01).

In order to illustrate the invention, the following examples are included. However, it is to be understood that these examples do not limit the invention and are only meant to suggest a method of practicing the invention.

Persons skilled in the art will recognize that the chemical reactions described may be readily adapted to prepare a number of other glucokinase activators of the invention, and alternative methods for preparing the compounds of this invention are deemed to be within the scope of this disclosure. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds of the invention.

Above and below, all temperatures are indicated in°C. In the following examples, "conventional work-up" means: if necessary, water is added, the pH is adjusted, if necessary, to between 2 and 10, depending on the constitution of the end product, the mixture is extracted with ethyl acetate or dichloromethane, the phases are separated, the organic phase is dried over sodium sulfate and evaporated, and the product is purified by chromatography on silica gel and/or by crystallisation. Rf values on silica gel; eluent: ethyl acetate/methanol 9:1.
Mass spectrometry (MS): EI (electron impact ionisation) M⁺ FAB (fast atom bombardment) (M+H)⁺ ESI (electrospray ionisation) (M+H)⁺ (unless indicated otherwise)
**Melting Points (mp.):** melting points are determined with a BÜCHI Melting Point B-540

### LC-MS-conditions

Mass data (MH⁺, given as m/z values) were taken from LC-MS measurements and were recorded with a Hewlett Packard System of the HP 1100 series with an ELS-detector Sedex 75 from ERC with the following characteristics: Ion source: Electrospray (positive mode); Scan: 100-1000 m/z; Fragmentation-voltage: 60 V; Gas-temperature: 300°C, DAD: 220 nm.
Flow rate: 2.4 ml/Min. The used splitter reduced the flow rate after the DAD for the MS to 0,75ml/Min.
Column: Chromolith Speed ROD RP-18e 50-4.6
Solvent: LiChrosolv (Merck KGaA)
Solvent A: H2O (0.01% TFA)
Solvent B: ACN (0.01% TFA)
Method A: In 2.6 min from 96% A to 100% B. Followed by 0.7 min 100% B.

### SFC-conditions for enantiomer separation

Berger SFC™ Minigram (tubing: preparative mode)
column: Chiralpak AS-H (Daicel), 5µm, 4.6 mm x 250 mm
eluent: method A: 85% CO₂/15% MeOH; method B: 70% CO₂/30% MeOH
flow: 5 ml/min
outlet pressure: 100 bar
column temperature: 35°C
UV: 250 nm
preparative injections: method A: 100µl of a 4mg/ml ACN/MeOH (1:1) solution; method B: 100µl of a 5mg/ml ACN/MeOH (3:2) solution

### Example 1

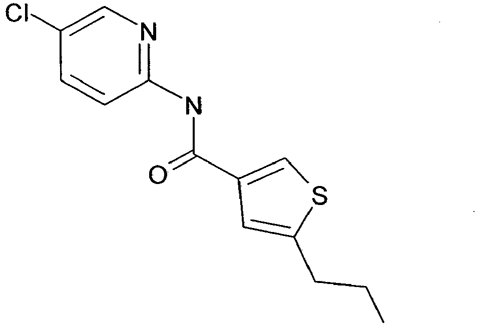

### 5-Propyl-thiophene-3-carboxylic acid (5-chloro-pyridin-2-yl)-amide

5-Propylthiophene-3-carboxylic acid (1.5 mmol), N-(3-Dimethylaminopropyl)-N'-ethylcarbodiimidhydrochlorid (1.1eq), 2-Amino-5-chlorpyridin (1.1 eq.) and 1-Hydroxybenzotriazolhydrat (1.1 eq) and 4-Methylmorpholin (1.6 eq.) are dissolved in DMF and stirred four days at room temperature. Water is added to the reaction solution and extracted with dichloromethane. The combined organic layers are washed with 1N NaOH and brine, dried over N₂SO₄ and the solvent is removed in vacuum. 5-Propyl-thiophene-3-carboxylic acid is obtained after column chromatography (Heptan / ethyl acetate) as colorless solid in a yield of 11%. HPLC (Method A): 3.56 min; LC-MS (Method A): 2.58 min, 281.00 (MH⁺); ¹H-NMR (DMSO-d6 500 MHz): δ [ppm] 10.715 (s, 1H), 8.42 (d, 1 H, J=2.6 Hz), 8.343 (d, 1H, J=1.1 Hz), 8.2 (d, 1 H, J=8.9 Hz), 7.933 (dd, 1H, J=2.6 Hz, J=8.9 Hz), 7.415 (d, 1H, J=1.1 Hz), 2.779 (t, 2H, J=7.4 Hz), 1.657 (sextett, 2H, J=7.4 Hz), 0.942 (t, 3H, J=7.4 Hz).

The following compounds can be synthesized via a similar reaction path as in example 1:

### Example 2

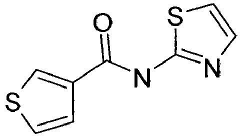

Thiophene-3-carboxylic acid thiazol-2-ylamide. beige solid, 48 % yield, HPLC (Method A): 2.81 min, LCMS (Method A): 1.59 min, 211 m/z (MH+)

### Example 3

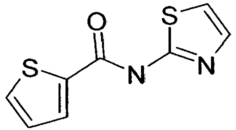

Thiophene-2-carboxylic acid thiazol-2-ylamide: beige solid, 23 % yield, HPLC (Method A): 2.83 min, LCMS (Method A): 1.61 min, 211 m/z (MH+)

### Example 4

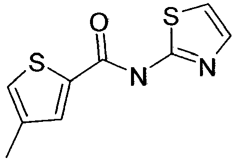

4-Methyl-thiophene-2-carboxylic acid thiazol-2-ylamide: beige solid, 51 % yield, HPLC (Method A): 2.99 min, LCMS (Method A): 1.83 min, 225 m/z (MH+)

### Example 5

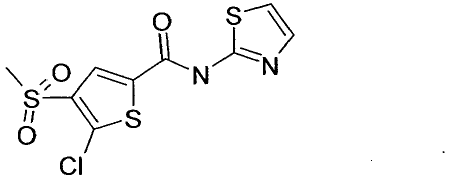

5-Chloro-4-methanesulfonyl-thiophene-2-carboxylic acid thiazol-2-ylamide: colorless solid, 4 % yield, HPLC (Method A): 2.92 min, LCMS (Method A): 1.72 min, 323 m/z (MH+)

### Example 6

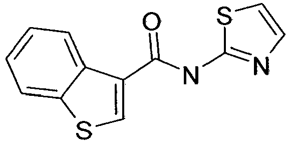

Benzo[b]thiophene-3-carboxylic acid thiazol-2-ylamide: colorless solid; 5 % yield, HPLC (Method A): 3.21 min, LCMS (Method A): 2.12 min, 261 m/z (MH+)

### Example 7

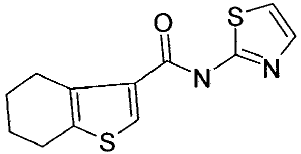

4,5,6,7-Tetrahydro-benzo[b]thiophene-3-carboxylic acid thiazol-2-ylamide: yellow solid, 8 % yield, HPLC (Method A): 3.31 min, LCMS (Method A): 2.26 min, 265 m/z (MH+)

### Example 8

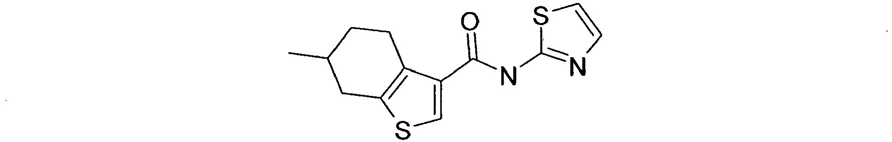

6-Methyl-4,5,6,7-tetrahydro-benzo[b]thiophene-3-carboxylic acid thiazol-2-ylamide: colorless solid, 10 % yield, HPLC (Method A): 3.47 min, LCMS (Method A): 2.46 min, 279.2 m/z (MH+)

### Example 9

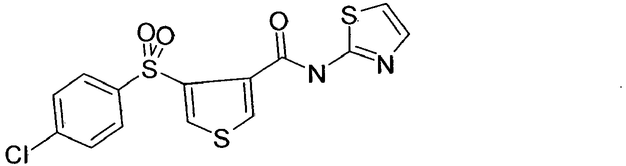

4-(4-Chloro-benzenesulfonyl)-thiophene-3-carboxylic acid thiazol-2-ylamide: colorless solid, 41 % yield, HPLC (Method A): 3.19 min, LCMS (Method A): 2.08 min, 385 m/z (MH+)

### Example 10

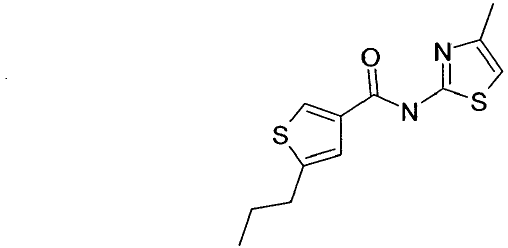

5-Propyl-thiophene-3-carboxylic acid (4-methyl-thiazol-2-yl)-amide: beige solid, 42 % yield, HPLC (Method A): 3.37 min, LCMS (Method A): 2.35 min, 267.2 m/z (MH+)

### Example 11

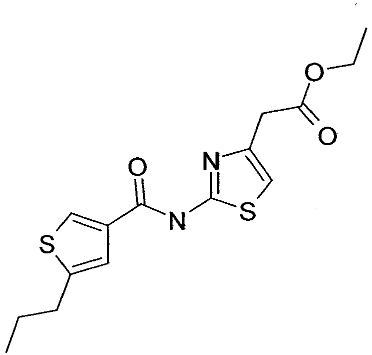

{2-[(5-Propyl-thiophene-3-carbonyl)-amino]-thiazol-4-yl}-acetic acid ethyl ester: brown oil, 16 % yield, HPLC (Method A): 3.47 min, LCMS (Method A): 2.42 min, 339.2 m/z (MH+)

### Example 12

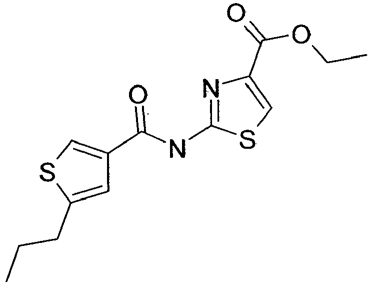

2-[(5-Propyl-thiophene-3-carbonyl)-amino]-thiazole-4-carboxylic acid ethyl ester: beige solid, 5 % yield, HPLC (Method A): 3.49 min, LCMS (Method A): 2.45 min, 325.2 m/z (MH+)

### Example 13

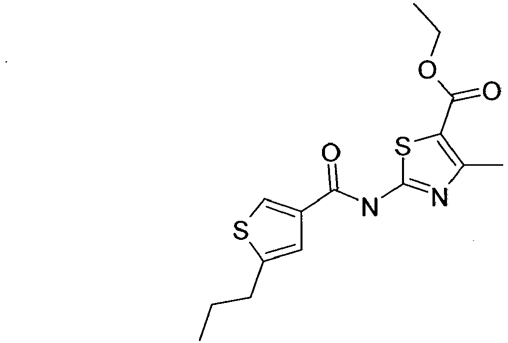

4-Methyl-2-[(5-propyl-thiophene-3-carbonyl)-amino]-thiazole-5-carboxylic acid ethyl ester: colorless solid, 39 % yield, HPLC (Method A): 3.64 min, LCMS (Method A): 2.66 min, 339.2 m/z (MH+)

### Example 14

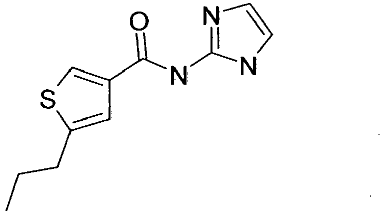

5-Propyl-thiophene-3-carboxylic acid (1H-imidazol-2-yl)-amide: beige solid, 36 % yield, HPLC (Method A): 2.84 min, LCMS (Method A): 1.46 min, 236.2 m/z (MH+)

### Example 15

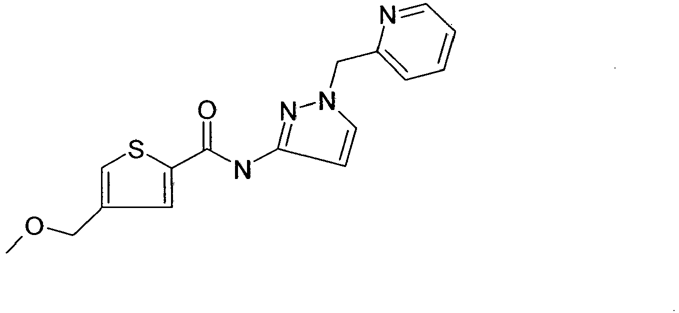

4-Methoxymethyl-thiophene-2-carboxylic acid (1-pyridin-2-ylmethyl-1H-pyrazol-3-yl)-amide: LCMS 329.2 m/z (MH+)

### Example 16

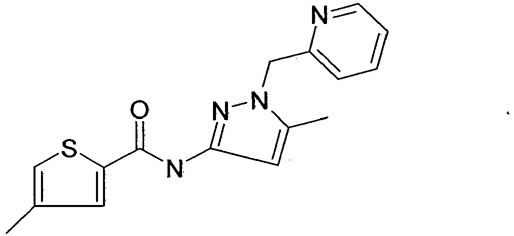

4-Methyl-thiophene-2-carboxylic acid (5-methyl-1-pyridin-2-ylmethyl-1H-pyrazol-3-yl)-amide: LCMS 313.1 m/z (MH+)

### Example 17

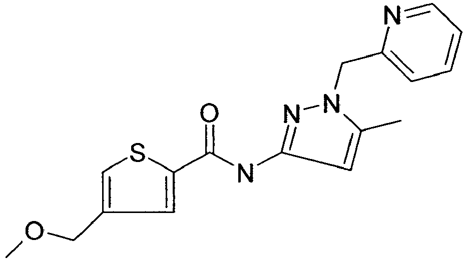

4-Methoxymethyl-thiophene-2-carboxylic acid (5-methyl-1-pyridin-2-ylmethyl-1H-pyrazol-3-yl)-amide: LCMS 343.2 m/z (MH+)

### Example 18

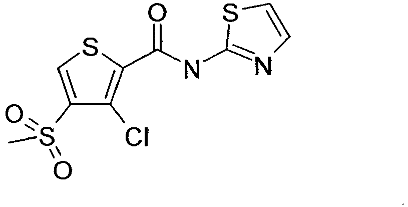

3-Chloro-4-methanesulfonyl-thiophene-2-carboxylic acid thiazol-2-ylamide: orange solid, 27 % yield, HPLC (Method A): 2.75 min, LCMS (Method A): 1.47 min, 323 m/z (MH+)

### Example 19

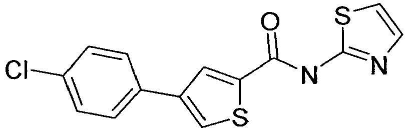

4-(4-Chloro-phenyl)-thiophene-2-carboxylic acid thiazol-2-ylamide: beige solid, 71 % yield, HPLC (Method A): 3.49 min, LCMS (Method A): 2.45 min, 321 m/z (MH+)

### Example 20

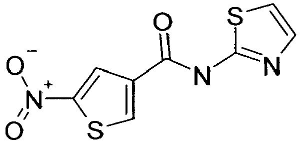

5-Nitro-thiophene-3-carboxylic acid thiazol-2-ylamide: brown solid, 36 % yield, HPLC (Method A): 2.97 min, LCMS (Method A): 1.79 min, 256 m/z (MH+)

### Example 21

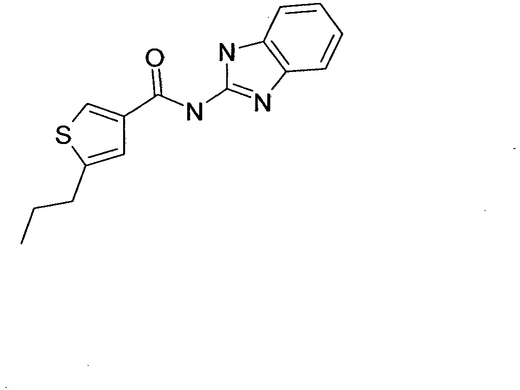

5-Propyl-thiophene-3-carboxylic acid (1 H-benzoimidazol-2-yl)-amide: beige solid, 37 % yield, HPLC (Method A): 3.12 min, LCMS (Method A): 1.81 min, 286.2 m/z (MH+)

### Example 22

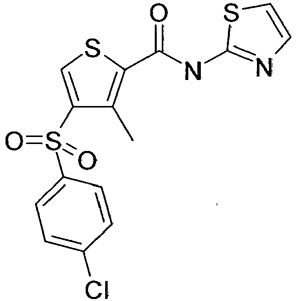

4-(4-Chloro-benzenesulfonyl)-3-methyl-thiophene-2-carboxylic acid thiazol-2-ylamide: colorless solid, 64 % yield, HPLC (Method A): 3.31 min, LCMS (Method A): 2.2 min, 399 m/z (MH+)

### Example 23

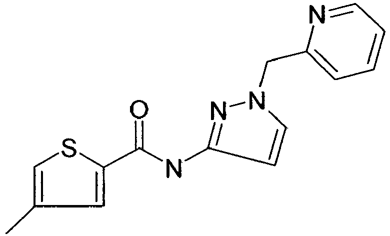

4-Methyl-thiophene-2-carboxylic acid (1-pyridin-2-ylmethyl-1H-pyrazol-3-yl)-amide: LCMS 291.1 m/z (MH+)

### Example 24

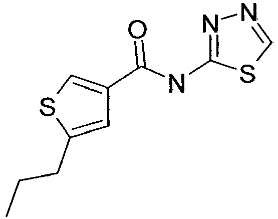

5-Propyl-thiophene-3-carboxylic acid [1,3,4]thiadiazol-2-ylamide: beige solid, 27 % yield, HPLC (Method A): 3.16 min, LCMS (Method A): 2.02 min, 254 m/z (MH+)

### Example 25

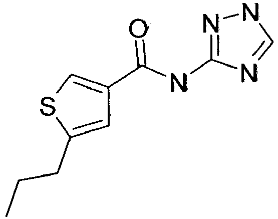

5-Propyl-thiophene-3-carboxylic acid (1 H-[1,2,4]triazol-3-yl)-amide: beige solid, 60 % yield, HPLC (Method A): 3.23 min, LCMS (Method A): 2.23 min, 237.2 m/z (MH+)

### Example 26

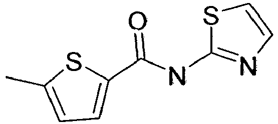

5-Methyl-thiophene-2-carboxylic acid thiazol-2-ylamide: brown solid, 56 % yield, HPLC (Method A): 2.96 min, LCMS (Method A): 1.82 min, 225 m/z (MH+)

### Example 27

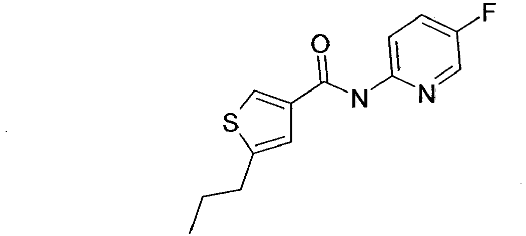

5-Propyl-thiophene-3-carboxylic acid (5-fluoro-pyridin-2-yl)-amide: yellow solid, 31 % yield, HPLC (Method A): 3.4 min, LCMS (Method A): 2.38 min, 265.2 m/z (MH+)

### Example 28

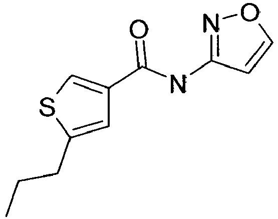

5-Propyl-thiophene-3-carboxylic acid isoxazol-3-ylamide: colorless solid, 16 % yield, HPLC (Method A): 3.21 min, LCMS (Method A): 2.11 min, 237.2 m/z (MH+)

### Example 29

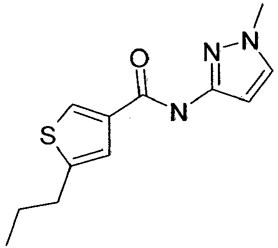

5-Propyl-thiophene-3-carboxylic acid (1-methyl-1 H-pyrazol-3-yl)-amide: colorless oil, 70 % yield, HPLC (Method A): 3.08 min, LCMS (Method A): 1.95 min, 250.2 m/z (MH+)

### Example 30

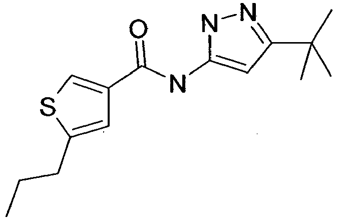

5-Propyl-thiophene-3-carboxylic acid (5-tert-butyl-2H-pyrazol-3-yl)-amide: colorless solid, 5 % yield, HPLC (Method A): 3.19 min, LCMS (Method A): 2.23 min, 292.2 m/z (MH+)

### Example 31

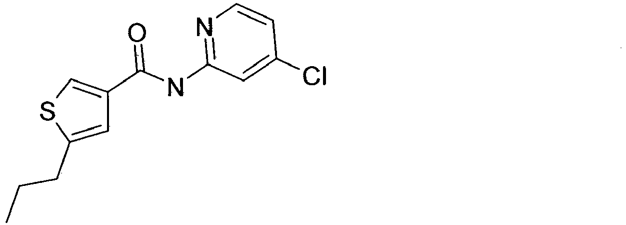

5-Propyl-thiophene-3-carboxylic acid (4-chloro-pyridin-2-yl)-amide: colorless oil, 4 % yield, HPLC (Method A): 3.41 min, LCMS (Method A): 2.58 min, 281 m/z (MH+)

### Example 32

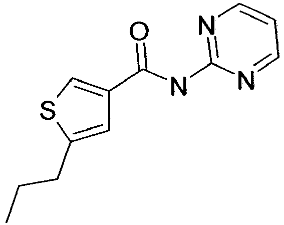

5-Propyl-thiophene-3-carboxylic acid pyrimidin-2-ylamide: yellow oil, 14 % yield, HPLC (Method A): 2.87 min, LCMS (Method A): 1.76 min, 248.2 m/z (MH+)

### Example 33

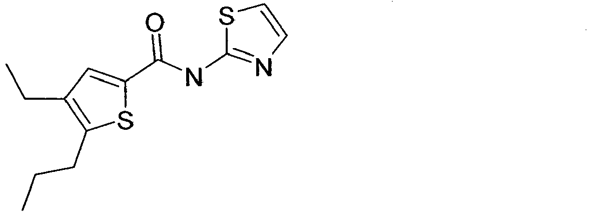

4-Ethyl-5-propyl-thiophene-2-carboxylic acid thiazol-2-ylamide: beige solid, 44 % yield, HPLC (Method A): 3.44 min, LCMS (Method A): 2.49 min, 281.2 m/z (MH+); ¹H-NMR (DMSO-d6, 300 MHz): δ [ppm] 12.489 (s, 1H), 8.078 (s, 1 H), 7.523 (d, 1H, J=3.5 Hz), 7.229 (d, 1H, J=3.5 Hz), 2.742 (t, 2H, J=7.6 Hz), 2.542 (t, 2H, J=7.6 Hz), 1.62 (sextett, 2H, J=7.6 Hz), 1.182 (t, 3H, J=7.6 Hz), 0.949 (t, 3H, J=7.2 Hz),

### Example 34

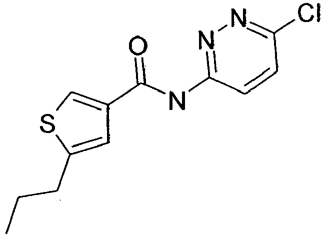

5-Propyl-thiophene-3-carboxylic acid (6-chloro-pyridazin-3-yl)-amide: colorless solid, 4 % yield, HPLC (Method A): 3.32 min, LCMS (Method A): 2.3 min, 282.2 m/z (MH+)

### Example 35

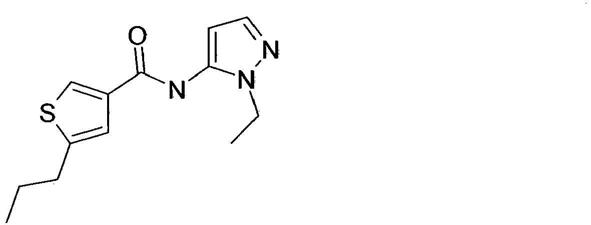

5-Propyl-thiophene-3-carboxylic acid (2-ethyl-2H-pyrazol-3-yl)-amide: colorless solid, 15 % yield, HPLC (Method A): 3.08 min, LCMS (Method A): 2 min, 246.2 m/z (MH+)

### Example 36

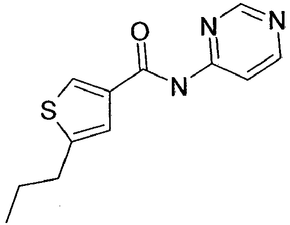

5-Propyl-thiophene-3-carboxylic acid pyrimidin-4-ylamide: yellow solid, 60 % yield, HPLC (Method A): 2.97 min, LCMS (Method A): 2.07 min, 248.2 m/z (MH+)

### Example 37

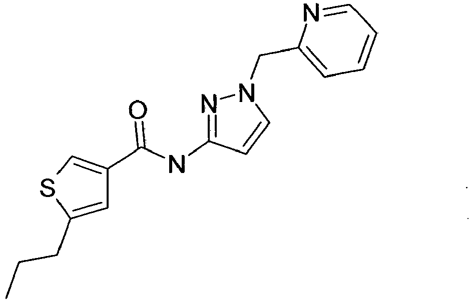

5-Propyl-thiophene-3-carboxylic acid (1-pyridin-2-ylmethyl-1 H-pyrazol-3-yl)-amide: beige solid, 86 % yield, HPLC (Method A): 2.89 min, LCMS (Method A): 1.91 min, 327.2 m/z (MH+)

### Example 38

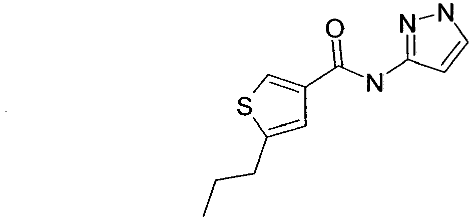

5-Propyl-thiophene-3-carboxylic acid (1H-pyrazol-3-yl)-amide: yellow solid, 2 % yield, HPLC (Method A): 2.95 min, LCMS (Method A): 1.82 min, 236.2 m/z (MH+)

### Example 39

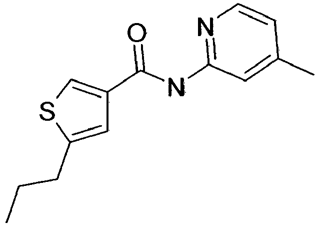

5-Propyl-thiophene-3-carboxylic acid (4-methyl-pyridin-2-yl)-amide: yellow oil, 10 % yield, HPLC (Method A): 2.89 min, LCMS (Method A): 1.79 min, 261.2 m/z (MH+)

### Example 40

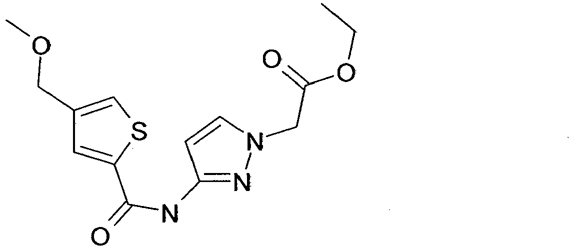

{3-[(4-Methoxymethyl-thiophene-2-carbonyl)-amino]-pyrazol-1-yl}-acetic acid ethyl ester: LCMS 324 m/z (MH+)

### Example 41

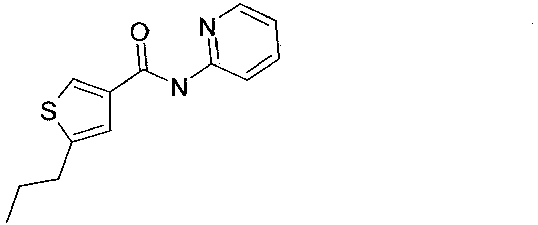

5-Propyl-thiophene-3-carboxylic acid pyridin-2-ylamide: yellow oil, 4 % yield, HPLC (Method A): 2.87 min, LCMS (Method A): 1.83 min, 247.2 m/z (MH+).

### Example 42

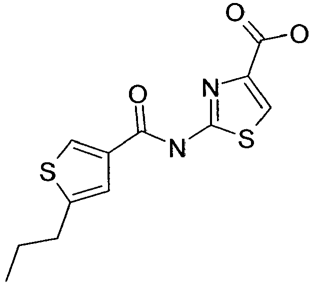

2-[(5-Propyl-thiophene-3-carbonyl)-amino]-thiazole-4-carboxylic acid 2-[(5-Propyl-thiophene-3-carbonyl)-amino]-thiazole-4-carboxylic acid ethyl ester (0.077 mmol) is dissolved in EtOH (0.5 ml) and 1 N NaOH (2.5 eq) is added. The reaction solution is stirred 20 hours at 40 °C. The pH is adjusted to 2 and the precipitate is filtered. 2-[(5-Propyl-thiophene-3-carbonyl)-amino]-thiazole-4-carboxylic acid is obtained as colorless solid in a yield of 61 %. HPLC (Method A): 3.2 min, LCMS (Method A): 2.05 min, 297 m/z (MH+)

The following compounds can be synthesized via a similar reaction path as in example 42:

### Example 43

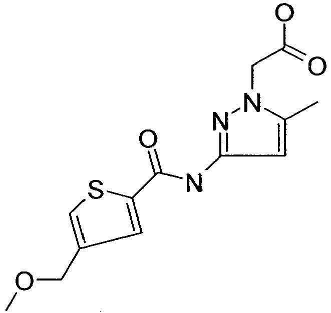

{3-[(4-Methoxymethyl-thiophene-2-carbonyl)-amino]-5-methyl-pyrazol-1-yl}-acetic acid: LCMS 310.1 m/z (MH+)

### Example 44

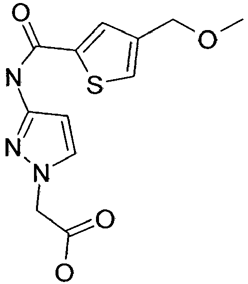

{3-[(4-Methoxymethyl-thiophene-2-carbonyl)-amino]-pyrazol-1-yl}-acetic acid: LCMS 296 m/z (MH+)

### Example 45

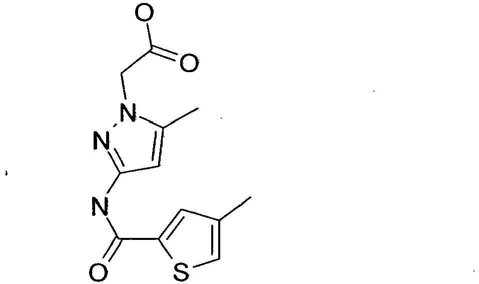

{5-Methyl-3-[(4-methyl-thiophene-2-carbonyl)-amino]-pyrazol-1-yl}-acetic acid: LCMS 280 m/z (MH+)

### Example 46

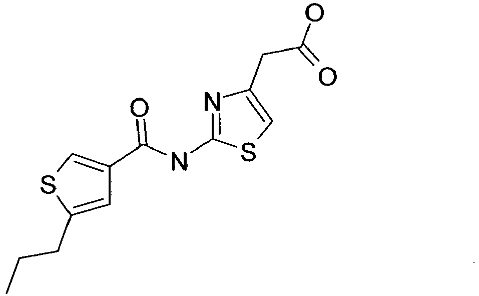

{2-[(5-Propyl-thiophene-3-carbonyl)-amino]-thiazol-4-yl}-acetic acid: colorless solid, 79 % yield, HPLC (Method A): 3.16 min, LCMS (Method A): 2.03 min, 311 m/z (MH+)

### Example 47

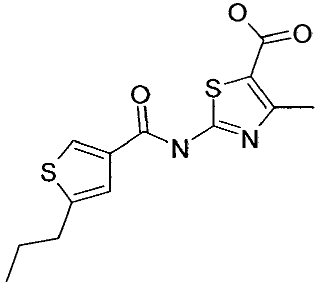

4-Methyl-2-[(5-propyl-thiophene-3-carbonyl)-amino]-thiazole-5-carboxylic acid: colorless solid, 87 % yield, HPLC (Method A): 3.12 min, LCMS (Method A): 1.9 min, 311 m/z (MH+)

### Example 48

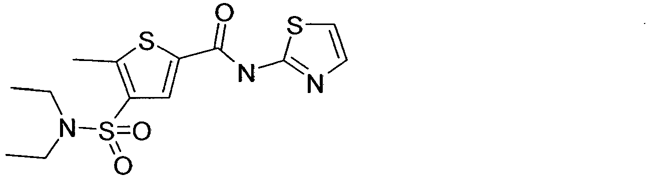

4-Diethylsulfamoyl-5-methyl-thiophene-2-carboxylic acid thiazol-2-ylamide
**Step A:** 5-Methyl-thiophene-2-carboxylic acid (30.5 mmol) is dissolved in Ethanol (53 ml), H2SO4 (0.5 eq) is added and the reaction is heated to reflux for 3 days. The solvent is removed in vacuo and the remaining material dissolved in Dichloromethane. The organic layer is extracted with saturated NaHCO₃ and washed with brine. The organic layer is dried over Na₂SO₄ and the solvent removed in vacuo. 5-Methylthiophene-2-carboxylic acid ethyl ester is obtained as brown oil in a yield of 67 %. HPLC (Method A): 3.36 min, LCMS (Method A): 2.20 min, 171.2 m/z (MH+)
**Step B:** 5-Methyl-thiophene-2-carboxylic acid ethyl ester (11.7 mmol) is dissolved in Chlorosulfonic acid (4 ml) at -4 °C and stirred 3 hours. The reaction solution is diluted with dichloromethane and ice water is added. The organic layer is washed with water, dried over MgSO₄ and the solvent is removed in vacuo. 4-Chlorosulfonyl-5-methyl-thiophene-2-carboxylic acid ethyl ester is obtained as brown oil in a yield of 35 %. HPLC (Method A): 3.57 min, LCMS (Method A): 2.48 min, 269.0 m/z (MH+)
**Step C:** 4-Chlorosulfonyl-5-methyl-thiophene-2-carboxylic acid ethyl ester (0.9 mmol) is dissolved in Dichloromethane (1.5 ml) and a suspension of Diethethylamin (1.1 eq.), Sodium acetate (2 eq) in Dichloromethane (1 ml) is added. The reaction is stirred 3 hours at room temperature. After addition of water, the reaction is extracted with Dichloromethane. 4-Diethylsulfamoyl-5-methyl-thiophene-2-carboxylic acid ethyl ester is obtained after removal of the organic solvent in vacuum as yello oil in a yield of 32 %. HPLC (Method A): 3.47 min, LCMS (Method A): 2.37 min, 306.2 m/z (MH+)
**Step D:** 4-Diethylsulfamoyl-5-methyl-thiophene-2-carboxylic acid ethyl ester (0.29 mmol) is dissolved in EtOH (0.5 ml) and 1 N NaOH (2.5 eq) is added. The reaction solution is stirred 24 hours at 40 °C. The pH is adjusted to 2 and the precipitate is filtered. 4-Diethylsulfamoyl-5-methylthiophene-2-carboxylic acid is obtained as colorless solid in a yield of 84 %. HPLC (Method A): 3.03 min, LCMS (Method A): 1.81 min, 278.2 m/z (MH+)
**Step E:** 4-Diethylsulfamoyl-5-methyl-thiophene-2-carboxylic acid (0.25 mmol), N-(3-Dimethylaminopropyl)-N'-ethylcarbodiimidhydrochlorid (1.1eq), 2-Amino-thiazol (1.1 eq.) and 1-Hydroxybenzotriazolhydrat (1.1 eq) and 4-Methylmorpholin (1.6 eq.) are dissolved in DMF and stirred four days at room temperature. Water is added to the reaction solution and extracted with dichloromethane. The combined organic layers are washed with 1N NaOH and brine, dried over N₂SO₄ and the solvent is removed in vacuum. 4-Diethylsulfamoyl-5-methyl-thiophene-2-carboxylic acid thiazol-2-ylamide is obtained after column chromatography (Heptan / ethyl acetate) as beige solid in a yield of 49 %. HPLC (Method A): 3.24 min, LCMS (Method A): 2.12 min, 360.1 m/z (MH+); ¹H-NMR (DMSO-d6, 500 MHz): d [ppm] 12.875 (br, 1 H), 8.371 (br, 1 H), 7.55 (d, 1H, J=3.6 Hz), 7.276 (br, 1H), 3.253 (q, 4H), 2.696 (s, 3H), 1.096 (t, 6H).

The following compounds can be synthesized via similar reactions as in example 48:

### Example 49

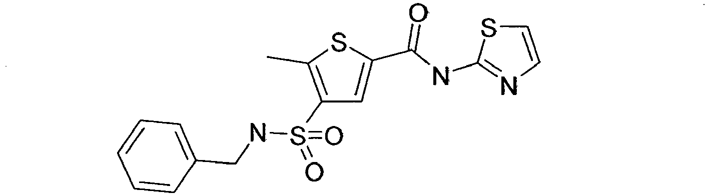

4-Benzylsulfamoyl-5-methyl-thiophene-2-carboxylic acid thiazol-2-ylamide: beige solid, HPLC (Method A): 3.24 min, LCMS (Method A): 2.08 min, 394 m/z (MH+)

### Example 50

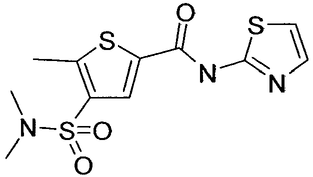

4-Dimethylsulfamoyl-5-methyl-thiophene-2-carboxylic acid thiazol-2-ylamide: beige solid, HPLC (Method A): 3.07 min, LCMS (Method A): 1.88 min, 332 m/z (MH+)

### Example 51

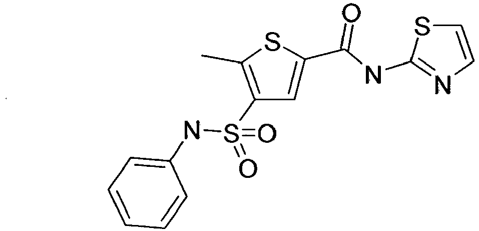

5-Methyl-4-phenylsulfamoyl-thiophene-2-carboxylic acid thiazol-2-ylamide: brown solid, HPLC (Method A): 3.2 min, LCMS (Method A): 2.07 min, 380.1 m/z (MH+)

### Example 52

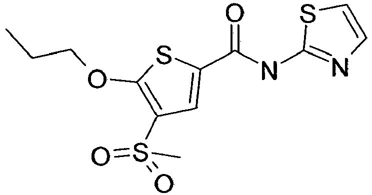

4-Methanesulfonyl-5-propoxy-thiophene-2-carboxylic acid thiazol-2-ylamide
**Step A:** 1-Propanol (1 mmol) is dissolved in THF (4 ml) and NaH (5.1 eq., 60% suspension in liquid paraffin) and 5-Chloro-4-methanesulfonyl-thiophene-2-carboxylic acid (1 eq.) is added. The suspension is stirred four hours at 60 °C and 15 hours at room temperature. After removal of the solvent in vacuo, the product is extracted with dichloromethane and water and the pH of the water phase is adjusted to 2 with 1 N HCl and extracted with dichloromethane. The organic layers are combined and the solvent removed in vacuo. 4-Methanesulfonyl-5-propoxy-thiophene-2-carboxylic acid is obtained as brown solid in a yield of 56 %. HPLC (Method A): 2.77 min, LCMS (Method A): 1.49 min, 265.0 m/z (MH+)
**Step B:** 4-Methanesulfonyl-5-propoxy-thiophene-2-carboxylic acid (0.5 mmol) is dissolved in Thionylchloride (1 ml) and THF (1 ml) and heated to 60 °C for 2 hours. The solvent is removed in vacuo. The remaininf solid is dissolved in Dichloromethane (0.75 ml) and Triethylamine (0.1 ml) and 2-Aminothiazole (1.5 eq.) is added. The suspension is stirred over night at room temperature. The precipitate is filtered and washed with Dichloromethane. 4-Methanesulfonyl-5-propoxy-thiophene-2-carboxylic acid thiazol-2-ylamide is obtained as beige solid in a yield of 51 %. HPLC (Method A): 3.00 min, LCMS (Method A): 1.87 min, 347 m/z (MH+); ¹H-NMR (DMSO-d6, 400 MHz): δ [ppm] 12.81 (s, 1H), 8.376 (s, 1H), 7.523 (d, 1H, J=3.5 Hz), 7.244 (d, 1H, J=3.5 Hz), 4.341 (t, 2H, J=6.3 Hz), 3.203 (s, 3H), 1.908-1.820 (m, 2H), 1.02 (t, 3H, J=7.5 Hz).

### Example 53

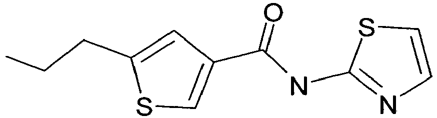

### 5-Propyl-thiophene-3-carboxylic acid thiazol-2-ylamide

### Example 54

### Pharmacological Data

**Table 1 Glucokinase Activation Assay**

| Example | hGK fold activation at 30 µM | hGK EC50 / µM |
|---|---|---|
| 1 | | |
| 2 | 1,3 | |
| 3 | 1,3 | |
| 4 | 3,4 | |
| 5 | 1,9 | 5,9 |
| 6 | 1,2 | |
| 7 | 1,2 | |
| 8 | 1,6 | |
| 9 | | |
| 10 | 5,2 | 9,3 |
| 11 | 4,9 | 15 |
| 12 | 2,6 | 5 |
| 13 | 1,5 | |
| 14 | 2,1 | |
| 15 | 3 | |
| 16 | 1,3 | |
| 17 | 1,8 | |
| 18 | 1,2 | |
| 19 | 1,5 | |
| 20 | 1,4 | |
| 21 | 2,4 | |
| 22 | | |
| 23 | 3,8 | |
| 24 | 2,4 | |
| 25 | | |
| 26 | 1,5 | |
| 27 | 2,1 | |
| 28 | 2 | |
| 29 | 5,5 | |
| 30 | | |
| 31 | 2,7 | |
| 32 | | |
| 33 | 9,4 | |
| 34 | | |
| 35 | 1,2 | |
| 36 | 2,7 | |
| 37 | 3,6 | |
| 38 | 2,7 | |
| 39 | 2,4 | |
| 40 | 2,6 | 4,2 |
| 41 | 2,5 | |
| 42 | 3,3 | 9 |
| 43 | 1,2 | |
| 44 | 1,7 | |
| 45 | | |
| 46 | 2,1 | |
| 47 | | |
| 48 | 6,4 | 3,9 |
| 49 | 1,3 | |
| 50 | 2,6 | 3,9 |
| 51 | | |
| 52 | 2,1 | 10 |
| 53 | 5,6 | 10 |

The following examples relate to pharmaceutical preparations:

### Example A: Injection vials

A solution of 100 g of an active ingredient according to the invention and 5 g of disodium hydrogenphosphate in 3 I of bidistilled water is adjusted to pH 6.5 using 2N hydrochloric acid, sterile filtered, transferred into injection vials, lyophilised under sterile conditions and sealed under sterile conditions. Each injection vial contains 5 mg of active ingredient.

### Example B: Suppositories

A mixture of 20 g of an active ingredient according to the invention with 100 g of soya lecithin and 1400 g of cocoa butter is melted, poured into moulds and allowed to cool. Each suppository contains 20 mg of active ingredient.

### Example C: Solution

A solution is prepared from 1 g of an active ingredient according to the invention, 9.38 g of NaH₂PO₄ · 2 H₂O, 28.48 g of Na₂HPO₄ 12 H₂O and 0.1 g of benzalkonium chloride in 940 ml of bidistilled water. The pH is adjusted to 6.8, and the solution is made up to 1 I and sterilised by irradiation. This solution can be used in the form of eye drops.

### Example D: Ointment

500 mg of an active ingredient according to the invention are mixed with 99.5 g of Vaseline under aseptic conditions.

### Example E: Tablets

A mixture of 1 kg of active ingredient according to the invention, 4 kg of lactose, 1.2 kg of potato starch, 0.2 kg of talc and 0.1 kg of magnesium stearate is pressed to give tablets in a conventional manner in such a way that each tablet contains 10 mg of active ingredient.

### Example F: Dragees

Tablets are pressed analogously to Example E and subsequently coated in a conventional manner with a coating of sucrose, potato starch, talc, tragacanth and dye.

### Example G: Capsules

2 kg of active ingredient according to the invention are introduced into hard gelatine capsules in a conventional manner in such a way that each capsule contains 20 mg of the active ingredient.

### Example H: Ampoules

A solution of 1 kg of an active ingredient according to the invention in 60 l of bidistilled water is sterile filtered, transferred into ampoules, lyophilised under sterile conditions and sealed under sterile conditions. Each ampoule contains 10 mg of active ingredient.

## Claims

1. Use of a compound selected from the group consisting of:
{3-[(4-Methoxymethyl-thiophene-2-carbonyl)-amino]-5-methyl-pyrazol-1-yl}-acetic acid, {3-[(4-Methoxymethyl-thiophene-2-carbonyl)-amino]-pyrazol-1-yl}-acetic acid, 4-Methyl-thiophene-2-carboxylic acid thiazol-2-ylamide, 5-Chloro-4-methanesulfonyl-thiophene-2-carboxylic acid thiazol-2-ylamide, {5-Methyl-3-[(4-methyl-thiophene-2-carbonyl)-amino]-pyrazol-1-yl}-acetic acid, 4-(4-Chloro-benzenesulfonyl)-thiophene-3-carboxylic acid thiazol-2-ylamide, 5-Propyl-thiophene-3-carboxylic acid (4-methyl-thiazol-2-yl)-amide, {2-[(5-Propyl-thiophene-3-carbonyl)-amino]-thiazol-4-yl}-acetic acid ethyl ester, 2-[(5-Propyl-thiophene-3-carbonyl)-amino]-thiazole-4-carboxylic acid ethyl ester, 4-Methyl-2-[(5-propyl-thiophene-3-carbonyl)-amino]-thiazole-5-carboxylic acid ethyl ester, 5-Propyl-thiophene-3-carboxylic acid (1 H-imidazol-2-yl)-amide, 4-Methoxymethyl-thiophene-2-carboxylic acid (1-pyridin-2-ylmethyl-1 H-pyrazol-3-yl)-amide, 4-Methyl-thiophene-2-carboxylic acid (5-methyl-1-pyridin-2-ylmethyl-1H-pyrazol-3-yl)-amide, 4-Methoxymethyl-thiophene-2-carboxylic acid (5-methyl-1-pyridin-2-ylmethyl-1 H-pyrazol-3-yl)-amide, 3-Chloro-4-methanesulfonyl-thiophene-2-carboxylic acid thiazol-2-ylamide, 4-(4-Chloro-phenyl)-thiophene-2-carboxylic acid thiazol-2-ylamide, 5-Nitro-thiophene-3-carboxylic acid thiazol-2-ylamide, {2-[(5-Propyl-thiophene-3-carbonyl)-amino]-thiazol-4-yl}-acetic acid, 5-Propyl-thiophene-3-carboxylic acid (1H-benzoimidazol-2-yl)-amide, 4-(4-Chlorobenzenesulfonyl)-3-methyl-thiophene-2-carboxylic acid thiazol-2-ylamide, 2-[(5-Propyl-thiophene-3-carbonyl)-amino]-thiazole-4-carboxylic acid, 4-Methylthiophene-2-carboxylic acid (1-pyridin-2-ylmethyl-1H-pyrazol-3-yl)-amide, 5-Propyl-thiophene-3-carboxylic acid [1, 3, 4]thiadiazol-2-ylamide, 5-Propyl-thiophene-3-carboxylic acid (1H-[1, 2, 4]triazol-3-yl)-amide, 5-Methylthiophene-2-carboxylic acid thiazol-2-ylamide, 4-Methyl-2-[(5-propyl-thiophene-3-carbonyl)-amino]-thiazole-5-carboxylic acid, 5-Propyl-thiophene-3-carboxylic acid (5-fluoro-pyridin-2-yl)-amide, , 5-Propyl-thiophene-3-carboxylic acid (1-methyl-1 H-pyrazol-3-yl)-amide, 5-Amino-thiophene-3-carboxylic acid thiazol-2-ylamide, 5-Propyl-thiophene-3-carboxylic acid (5-tert-butyl-2H-pyrazol-3-yl)-amide, 5-Propyl-thiophene-3-carboxylic acid (4-chloro-pyridin-2-yl)-amide, 5-Propyl-thiophene-3-carboxylic acid pyrimidin-2-ylamide, 4-Ethyl-5-propyl-thiophene-2-carboxylic acid thiazol-2-ylamide, 5-Propyl-thiophene-3-carboxylic acid (6-chloro-pyridazin-3-yl)-amide, 5-Propyl-thiophene-3-carboxylic acid (2-ethyl-2H-pyrazol-3-yl)-amide, 5-Propyl-thiophene-3-carboxylic acid pyrimidin-4-ylamide, 5-Propyl-thiophene-3-carboxylic acid (1-pyridin-2-ylmethyl-1 H-pyrazol-3-yl)-amide, 5-Propyl-thiophene-3-carboxylic acid (1 H-pyrazol-3-yl)-amide, 5-Propyl-thiophene-3-carboxylic acid (4-methyl-pyridin-2-yl)-amide, {3-[(4-Methoxymethyl-thiophene-2-carbonyl)-amino]-pyrazol-1-yl}-acetic acid ethyl ester, 5-Propyl-thiophene-3-carboxylic acid pyridin-2-ylamide, 4-Benzylsulfamoyl-5-methylthiophene-2-carboxylic acid thiazol-2-ylamide, 4-Diethylsulfamoyl-5-methylthiophene-2-carboxylic acid thiazol-2-ylamide, , 5-Methyl-4-phenylsulfamoyl-thiophene-2-carboxylic acid thiazol-2-ylamide, 4-Methanesulfonyl-5-propoxy-thiophene-2-carboxylic acid thiazol-2-ylamide
and/or salts, solvates and stereoisomers thereof, including mixtures thereof in all ratios, and optionally excipients and/or adjuvants,
for the preparation of a medicament for the treatment of insulin-dependent diabetes mellitus, non-insulin-dependent diabetes mellitus, obesity, neuropathy and/or nephropathy.

2. A medicament comprising at least one compound selected from the group consisting of:
{3-[(4-Methoxymethyl-thiophene-2-carbonyl)-amino]-5-methyl-pyrazol-1-yl}-acetic acid, {3-[(4-Methoxymethyl-thiophene-2-carbonyl)-amino]-pyrazol-1-yl}-acetic acid, 4-Methyl-thiophene-2-carboxylic acid thiazol-2-ylamide, 5-Chloro-4-methanesulfonyl-thiophene-2-carboxylic acid thiazol-2-ylamide, {5-Methyl-3-[(4-methyl-thiophene-2-carbonyl)-amino]-pyrazol-1-yl}-acetic acid, 4-(4-Chloro-benzenesulfonyl)-thiophene-3-carboxylic acid thiazol-2-ylamide, 5-Propyl-thiophene-3-carboxylic acid (4-methyl-thiazol-2-yl)-amide, {2-[(5-Propyl-thiophene-3-carbonyl)-amino]-thiazol-4-yl}-acetic acid ethyl ester, 2-[(5-Propyl-thiophene-3-carbonyl)-amino]-thiazole-4-carboxylic acid ethyl ester, 4-Methyl-2-[(5-propyl-thiophene-3-carbonyl)-amino]-thiazole-5-carboxylic acid ethyl ester, 5-Propyl-thiophene-3-carboxylic acid (1 H-imidazol-2-yl)-amide, 4-Methoxymethyl-thiophene-2-carboxylic acid (1-pyridin-2-ylmethyl-1H-pyrazol-3-yl)-amide, 4-Methyl-thiophene-2-carboxylic acid (5-methyl-1-pyridin-2-ylmethyl-1 H-pyrazol-3-yl)-amide, 4-Methoxymethyl-thiophene-2-carboxylic acid (5-methyl-1-pyridin-2-ylmethyl-1 H-pyrazol-3-yl)-amide, 3-Chloro-4-methanesulfonyl-thiophene-2-carboxylic acid thiazol-2-ylamide, 4-(4-Chloro-phenyl)-thiophene-2-carboxylic acid thiazol-2-ylamide, 5-Nitro-thiophene-3-carboxylic acid thiazol-2-ylamide, {2-[(5-Propyl-thiophene-3-carbonyl)-amino]-thiazol-4-yl}-acetic acid, 5-Propyl-thiophene-3-carboxylic acid (1 H-benzoimidazol-2-yl)-amide, 4-(4-Chlorobenzenesulfonyl)-3-methyl-thiophene-2-carboxylic acid thiazol-2-ylamide, 2-[(5-Propyl-thiophene-3-carbonyl)-amino]-thiazole-4-carboxylic acid, 4-Methylthiophene-2-carboxylic acid (1-pyridin-2-ylmethyl-1 H-pyrazol-3-yl)-amide, 5-Propyl-thiophene-3-carboxylic acid [1, 3, 4]thiadiazol-2-ylamide, 5-Propyl-thiophene-3-carboxylic acid (1 H-[1, 2, 4]triazol-3-yl)-amide, 5-Methylthiophene-2-carboxylic acid thiazol-2-ylamide, 4-Methyl-2-[(5-propyl-thiophene-3-carbonyl)-amino]-thiazole-5-carboxylic acid, 5-Propyl-thiophene-3-carboxylic acid (5-fluoro-pyridin-2-yl)-amide, , 5-Propyl-thiophene-3-carboxylic acid (1-methyl-1 H-pyrazol-3-yl)-amide, 5-Amino-thiophene-3-carboxylic acid thiazol-2-ylamide, 5-Propyl-thiophene-3-carboxylic acid (5-tert-butyl-2H-pyrazol-3-yl)-amide, 5-Propyl-thiophene-3-carboxylic acid (4-chloro-pyridin-2-yl)-amide, 5-Propyl-thiophene-3-carboxylic acid pyrimidin-2-ylamide, 4-Ethyl-5-propyl-thiophene-2-carboxylic acid thiazol-2-ylamide, 5-Propyl-thiophene-3-carboxylic acid (6-chloro-pyridazin-3-yl)-amide, 5-Propyl-thiophene-3-carboxylic acid (2-ethyl-2H-pyrazol-3-yl)-amide, 5-Propyl-thiophene-3-carboxylic acid pyrimidin-4-ylamide, 5-Propyl-thiophene-3-carboxylic acid (1-pyridin-2-ylmethyl-1 H-pyrazol-3-yl)-amide, 5-Propyl-thiophene-3-carboxylic acid (1 H-pyrazol-3-yl)-amide, 5-Propyl-thiophene-3-carboxylic acid (4-methyl-pyridin-2-yl)-amide, {3-[(4-Methoxymethyl-thiophene-2-carbonyl)-amino]-pyrazol-1-yl}-acetic acid ethyl ester, 5-Propyl-thiophene-3-carboxylic acid pyridin-2-ylamide, 4-Benzylsulfamoyl-5-methylthiophene-2-carboxylic acid thiazol-2-ylamide, 4-Diethylsulfamoyl-5-methylthiophene-2-carboxylic acid thiazol-2-ylamide, , 5-Methyl-4-phenylsulfamoyl-thiophene-2-carboxylic acid thiazol-2-ylamide, 4-Methanesulfonyl-5-propoxy-thiophene-2-carboxylic acid thiazol-2-ylamide,
and/or salts, solvates and stereoisomers thereof, including mixtures thereof in all ratios, and optionally excipients and/or adjuvants.

## Patentansprüche

1. Verwendung einer Verbindung, die ausgewählt ist aus der Gruppe bestehend aus:
{3-[(4-Methoxymethyl-thiophen-2-carbonyl)-amino]-5-methyl-pyrazol-1-yl}-essigsäure, {3-[(4-Methoxymethyl-thiophen-2-carbonyl)-amino]-pyrazol-1-yl}-essigsäure, 4-Methyl-thiophen-2-carbonsäure-thiazol-2-ylamid, 5-Chlor-4-methansulfonyl-thiophen-2-carbonsäure-thiazol-2-ylamid, {5-Methyl-3-[(4-methyl-thiophen-2-carbonyl)-amino]-pyrazol-1-yl}-essigsäure, 4-(4-Chlor-benzolsulfonyl)-thiophen-3-carbonsäure-thiazol-2-ylamid, 5-Propyl-thiophen-3-carbonsäure-(4-methyl-thiazol-2-yl)-amid, {2-[(5-Propyl-thiophen-3-carbonyl)-amino]-thiazol-4-yl}-essigsäureethylester, 2-[(5-Propyl-thiophen-3-carbonyl)-amino]-thiazol-4-carbonsäureethylester, 4-Methyl-2-[(5-propyl-thiophen-3-carbonyl)-amino]-thiazol-5-carbonsäureethylester, 5-Propyl-thiophen-3-carbonsäure-(1H-imidazol-2-yl)-amid, 4-Methoxymethyl-thiophen-2-carbonsäure-(1-pyridin-2-ylmethyl-1H-pyrazol-3-yl)-amid, 4-Methyl-thiophen-2-carbonsäure-(5-methyl-1-pyridin-2-ylmethyl-1 H-pyrazol-3-yl)-amid, 4-Methoxymethyl-thiophen-2-carbonsäure-(5-methyl-1-pyridin-2-ylmethyl-1 H-pyrazol-3-yl)-amid, 3-Chlor-4-methansulfonyl-thiophen-2-carbonsäure-thiazol-2-ylamid, 4-(4-Chlor-phenyl)-thiophen-2-carbonsäure-thiazol-2-ylamid, 5-Nitro-thiophen-3-carbonsäure-thiazol-2-ylamid, {2-[(5-Propyl-thiophen-3-carbonyl)-amino]-thiazol-4-yl}-essigsäure, 5-Propyl-thiophen-3-carbonsäure-(1H-benzoimidazol-2-yl)-amid, 4-(4-Chlor-benzol-sulfonyl)-3-methyl-thiophen-2-carbonsäure-thiazol-2-ylamid, 2-[(5-Propyl-thiophen-3-carbonyl)-amino]-thiazol-4-carbonsäure, 4-Methyl-thiophen-2-carbonsäure-(1-pyridin-2-ylmethyl-1 H-pyrazol-3-yl)-amid, 5-Propyl-thiophen-3-carbonsäure[1,3,4]thiadiazol-2-ylamid, 5-Propyl-thiophen-3-carbonsäure-(1H-[1,2,4]triazol-3-yl)-amid, 5-Methyl-thiophen-2-carbonsäure-thiazol-2-ylamid, 4-Methyl-2-[(5-propyl-thiophen-3-carbonyl)-amino]-thiazol-5-carbonsäure, 5-Propyl-thiophen-3-carbonsäure-(5-fluor-pyridin-2-yl)-amid, 5-Propyl-thiophen-3-carbonsäure-(1-methyl-1H-pyrazol-3-yl)-amid, 5-Amino-thiophen-3-carbonsäure-thiazol-2-ylamid, 5-Propyl-thiophen-3-carbonsäure-(5-tert-butyl-2H-pyrazol-3-yl)-amid, 5-Propyl-thiophen-3-carbonsäure-(4-chlor-pyridin-2-yl)-amid, 5-Propyl-thiophen-3-carbonsäure-pyrimidin-2-ylamid, 4-Ethyl-5-propyl-thiophen-2-carbonsäure-thiazol-2-ylamid, 5-Propyl-thiophen-3-carbonsäure-(6-chlor-pyridazin-3-yl)-amid, 5-Propyl-thiophen-3-carbonsäure-(2-ethyl-2H-pyrazol-3-yl)-amid, 5-Propyl-thiophen-3-carbonsäure-pyrimidin-4-ylamid, 5-Propyl-thiophen-3-carbonsäure-(1-pyridin-2-ylmethyl-1H-pyrazol-3-yl)-amid, 5-Propyl-thiophen-3-carbonsäure-(1H-pyrazol-3-yl)-amid, 5-Propyl-thiophen-3-carbonsäure-(4-methyl-pyridin-2-yl)-amid, {3-[(4-Methoxymethyl-thiophen-2-carbonyl)-amino]-pyrazol-1-yl}-essigsäureethylester, 5-Propyl-thiophen-3-carbonsäure-pyridin-2-ylamid, 4-Benzylsulfamoyl-5-methyl-thiophen-2-carbonsäure-thiazol-2-ylamid, 4-Diethylsulfamoyl-5-methyl-thiophen-2-carbonsäure-thiazol-2-ylamid, 5-Methyl-4-phenylsulfamoyl-thiophen-2-carbonsäure-thiazol-2-ylamid, 4-Methansulfonyl-5-propoxy-thiophen-2-carbonsäure-thiazol-2-ylamid
und/oder Salzen, Solvaten und Stereoisomeren davon, einschließlich deren Mischungen in allen Verhältnissen, und gegebenenfalls Streckmitteln und/oder Adjuvantien,
zur Herstellung eines Arzneimitteln für die Behandlung von insulinabhängigem Diabetes mellitus, nicht insulinabhängigem Diabetes mellitus, Fettsucht, Neuropathie und/oder Nephropathie.

2. Arzneimittel enthaltend mindestens eine Verbindung, die ausgewählt ist aus der Gruppe bestehend aus:
{3-[(4-Methoxymethyl-thiophen-2-carbonyl)-amino]-5-methyl-pyrazol-1-yl}-essigsäure, {3-[(4-Methoxymethyl-thiophen-2-carbonyl)-amino]-pyrazol-1-yl}-essigsäure, 4-Methyl-thiophen-2-carbonsäure-thiazol-2-ylamid, 5-Chlor-4-methansulfonyl-thiophen-2-carbonsäure-thiazol-2-ylamid, {5-Methyl-3-[(4-methyl-thiophen-2-carbonyl)-amino]-pyrazol-1-yl}-essigsäure, 4-(4-Chlor-benzolsulfonyl)-thiophen-3-carbonsäure-thiazol-2-ylamid, 5-Propyl-thiophen-3-carbonsäure-(4-methyl-thiazol-2-yl)-amid, {2-[(5-Propyl-thiophen-3-carbonyl)-amino]-thiazol-4-yl}-essigsäureethylester, 2-[(5-Propyl-thiophen-3-carbonyl)-amino]-thiazol-4-carbonsäureethylester, 4-Methyl-2-[(5-propyl-thiophen-3-carbonyl)-amino]-thiazol-5-carbonsäureethylester, 5-Propyl-thiophen-3-carbonsäure-(1H-imidazol-2-yl)-amid, 4-Methoxymethyl-thiophen-2-carbonsäure-(1-pyridin-2-ylmethyl-1H-pyrazol-3-yl)-amid, 4-Methyl-thiophen-2-carbonsäure-(5-methyl-1-pyridin-2-ylmethyl-1 H-pyrazol-3-yl)-amid, 4-Methoxymethyl-thiophen-2-carbonsäure-(5-methyl-1-pyridin-2-yl-methyl-1 H-pyrazol-3-yl)-amid, 3-Chlor-4-methansulfonyl-thiophen-2-carbonsäure-thiazol-2-ylamid, 4-(4-Chlor-phenyl)-thiophen-2-carbonsäure-thiazol-2-ylamid, 5-Nitro-thiophen-3-carbonsäure-thiazol-2-ylamid, {2-[(5-Propyl-thiophen-3-carbonyl)-amino]-thiazol-4-yl}-essigsäure, 5-Propyl-thiophen-3-carbonsäure-(1H-benzoimidazol-2-yl)-amid, 4-(4-Chlor-benzolsulfonyl)-3-methyl-thiophen-2-carbonsäure-thiazol-2-ylamid, 2-[(5-Propyl-thiophen-3-carbonyl)-amino]-thiazol-4-carbonsäure, 4-Methyl-thiophen-2-carbonsäure-(1-pyridin-2-ylmethyl-1 H-pyrazol-3-yl)-amid, 5-Propyl-thiophen-3-carbonsäure[1,3,4]thiadiazol-2-ylamid, 5-Propyl-thiophen-3-carbonsäure-(1H-[1,2,4]-triazol-3-yl)-amid, 5-Methyl-thiophen-2-carbonsäure-thiazol-2-ylamid, 4-Methyl-2-[(5-propyl-thiophen-3-carbonyl)-amino]-thiazol-5-carbonsäure, 5-Propyl-thiophen-3-carbonsäure-(5-fluor-pyridin-2-yl)-amid, 5-Propyl-thiophen-3-carbonsäure-(1-methyl-1H-pyrazol-3-yl)-amid, 5-Amino-thiophen-3-carbonsäure-thiazol-2-ylamid, 5-Propyl-thiophen-3-carbonsäure-(5-tert-butyl-2H-pyrazol-3-yl)-amid, 5-Propyl-thiophen-3-carbonsäure-(4-chlor-pyridin-2-yl)-amid, 5-Propyl-thiophen-3-carbonsäure-pyrimidin-2-ylamid, 4-Ethyl-5-propyl-thiophen-2-carbonsäure-thiazol-2-ylamid, 5-Propyl-thiophen-3-carbonsäure-(6-chlor-pyridazin-3-yl)-amid, 5-Propyl-thiophen-3-carbonsäure-(2-ethyl-2H-pyrazol-3-yl)-amid, 5-Propyl-thiophen-3-carbonsäure-pyrimidin-4-ylamid, 5-Propyl-thiophen-3-carbonsäure-(1-pyridin-2-ylmethyl-1H-pyrazol-3-yl)-amid, 5-Propyl-thiophen-3-carbonsäure-(1 H-pyrazol-3-yl)-amid, 5-Propyl-thiophen-3-carbonsäure-(4-methyl-pyridin-2-yl)-amid, {3-[(4-Methoxymethyl-thiophen-2-carbonyl)-amino]-pyrazol-1-yl}-essigsäureethylester, 5-Propyl-thiophen-3-carbonsäure-pyridin-2-ylamid, 4-Benzylsulfamoyl-5-methyl-thiophen-2-carbonsäure-thiazol-2-ylamid, 4-Diethylsulfamoyl-5-methyl-thiophen-2-carbonsäure-thiazol-2-ylamid, 5-Methyl-4-phenylsulfamoyl-thiophen-2-carbonsäure-thiazol-2-ylamid, 4-Methansulfonyl-5-propoxy-thiophen-2-carbonsäure-thiazol-2-ylamid,
und/oder Salze, Solvate und Stereoisomere davon, einschließlich deren Mischungen in allen Verhältnissen, und gegebenenfalls Streckmittel und/oder Adjuvantien.

## Revendications

1. Utilisation d'un composé choisi dans le groupe constitué par :
l'acide {3-[(4-méthoxyméthyl-thiophène-2-carbonyl)-amino]-5-méthyl-pyrazol-1-yl}-acétique, l'acide {3-[(4-méthoxyméthyl-thiophène-2-carbonyl)-amino]-pyrazol-1-yl}-acétique, l'acide 4-méthyl-thiophène-2-carboxylique thiazol-2-ylamide, l'acide 5-chloro-4-méthanesulfonyl-thiophène-2-carboxylique thiazol-2-ylamide, l'acide {5-méthyl-3-[(4-méthyl-thiophène-2-carbonyl)-amino]-pyrazol-1-yl}-acétique, l'acide 4-(4-chlorobenzènesulfonyl)-thiophène-3-carboxylique thiazol-2-ylamide, l'acide 5-propyl-thiophène-3-carboxylique (4-méthyl-thiazol-2-yl)-amide, l'acide {2-[(5-propyl-thiophène-3-carbonyl)-amino]-thiazol-4-yl}-acétique éthylester, l'acide 2-[(5-propyl-thiophène-3-carbonyl)-amino]-thiazole-4-carboxylique éthylester, l'acide 4-méthyl-2-[(5-propyl-thiophène-3-carbonyl)-amino]-thiazole-5-carboxylique éthylester, l'acide 5-propyl-thiophène-3-carboxylique (1H-imidazol-2-yl)-amide, l'acide 4-méthoxyméthyl-thiophène-2-carboxylique (1-pyridin-2-ylméthyl-1 H-pyrazol-3-yl)-amide, l'acide 4-méthyl-thiophène-2-carboxylique (5-méthyl-1-pyridin-2-ylméthyl-1H-pyrazol-3-yl)-amide, l'acide 4-méthoxyméthyl-thiophène-2-carboxylique (5-méthyl-1-pyridin-2-ylméthyl-1 H-pyrazol-3-yl)-amide, l'acide 3-chloro-4-méthanesulfonyl-thiophène-2-carboxylique thiazol-2-ylamide, l'acide 4-(4-chlorophényl)-thiophène-2-carboxylique thiazol-2-ylamide, l'acide 5-nitro-thiophène-3-carboxylique thiazol-2-ylamide, l'acide {2-[(5-propyl-thiophène-3-carbonyl)-amino]-thiazol-4-yl}-acétique, l'acide 5-propyl-thiophène-3-carboxylique (1 H-benzoimidazol-2-yl)-amide, l'acide 4-(4-chlorobenzène-sulfonyl)-3-méthyl-thiophène-2-carboxylique thiazol-2-ylamide, l'acide 2-[(5-propyl-thiophène-3-carbonyl)-amino]-thiazole-4-carboxylique, l'acide 4-méthyl-thiophène-2-carboxylique (1-pyridin-2-ylméthyl-1H-pyrazol-3-yl)-amide, l'acide 5-propyl-thiophène-3-carboxylique [1,3,4]thiadiazol-2-ylamide, l'acide 5-propyl-thiophène-3-carboxylique (1 H-[1,2,4]triazol-3-yl)-amide, l'acide 5-méthyl-thiophène-2-carboxylique thiazol-2-ylamide, l'acide 4-méthyl-2-[(5-propyl-thiophène-3-carbonyl)-amino]-thiazole-5-carboxylique, l'acide 5-propyl-thiophène-3-carboxylique (5-fluoro-pyridin-2-yl)-amide, l'acide 5-propyl-thiophène-3-carboxylique (1-méthyl-1 H-pyrazol-3-yl)-amide, l'acide 5-amino-thiophène-3-carboxylique thiazol-2-ylamide, l'acide 5-propyl-thiophène-3-carboxylique (5-tertio-butyl-2H-pyrazol-3-yl)-amide, l'acide 5-propyl-thiophène-3-carboxylique (4-chloro-pyridin-2-yl)-amide, l'acide 5-propyl-thiophène-3-carboxylique pyrimidin-2-ylamide, l'acide 4-éthyl-5-propyl-thiophène-2-carboxylique thiazol-2-ylamide, l'acide 5-propyl-thiophène-3-carboxylique (6-chloro-pyridazin-3-yl)-amide, l'acide 5-propyl-thiophène-3-carboxylique (2-éthyl-2H-pyrazol-3-yl)-amide, l'acide 5-propyl-thiophène-3-carboxylique pyrimidin-4-ylamide, l'acide 5-propyl-thiophène-3-carboxylique (1-pyridin-2-ylméthyl-1 H-pyrazol-3-yl)-amide, l'acide 5-propyl-thiophène-3-carboxylique (1 H-pyrazol-3-yl)-amide, l'acide 5-propyl-thiophène-3-carboxylique (4-méthyl-pyridin-2-yl)-amide, l'acide {3-[(4-méthoxyméthyl-thiophène-2-carbonyl)-amino]-pyrazol-1-yl}-acétique éthylester, l'acide 5-propyl-thiophène-3-carboxylique pyridin-2-ylamide, l'acide 4-benzylsulfamoyl-5-méthyl-thiophène-2-carboxylique thiazol-2-ylamide, l'acide 4-diéthylsulfamoyl-5-méthyl-thiophène-2-carboxylique thiazol-2-ylamide, l'acide 5-méthyl-4-phényl-sulfamoyl-thiophène-2-carboxylique thiazol-2-ylamide, l'acide 4-méthane-sulfonyl-5-propoxy-thiophène-2-carboxylique thiazol-2-ylamide,
et/ou les sels, solvats et stéréoisomères de ceux-ci, y compris des mélanges de ceux-ci en toutes proportions, et éventuellement des excipients et/ou des adjuvants,
pour la préparation d'un médicament destiné au traitement du diabète sucré insulinodépendant, du diabète sucré non insulinodépendant, de l'obésité, de la neuropathie et/ou de la néphropathie.

2. Médicament comprenant au moins un composé choisi dans le groupe constitué par:
l'acide {3-[(4-méthoxyméthyl-thiophène-2-carbonyl)-amino]-5-méthyl-pyrazol-1-yl}-acétique, l'acide {3-[(4-méthoxyméthyl-thiophène-2-carbonyl)-amino]-pyrazol-1-yl}-acétique, l'acide 4-méthyl-thiophène-2-carboxylique thiazol-2-ylamide, l'acide 5-chloro-4-méthanesulfonyl-thiophène-2-carboxylique thiazol-2-ylamide, l'acide {5-méthyl-3-[(4-méthyl-thiophène-2-carbonyl)-amino]-pyrazol-1-yl}-acétique, l'acide 4-(4-chlorobenzènesulfonyl)-thiophène-3-carboxylique thiazol-2-ylamide, l'acide 5-propyl-thiophène-3-carboxylique (4-méthyl-thiazol-2-yl)-amide, l'acide {2-[(5-propyl-thiophène-3-carbonyl)-amino]-thiazol-4-yl}-acétique éthylester, l'acide 2-[(5-propyl-thiophène-3-carbonyl)-amino]-thiazole-4-carboxylique éthylester, l'acide 4-méthyl-2-[(5-propyl-thiophène-3-carbonyl)-amino]-thiazole-5-carboxylique éthylester, l'acide 5-propyl-thiophène-3-carboxylique (1H-imidazol-2-yl)-amide, l'acide 4-méthoxyméthyl-thiophène-2-carboxylique (1-pyridin-2-ylméthyl-1H-pyrazol-3-yl)-amide, l'acide 4-méthyl-thiophène-2-carboxylique (5-méthyl-1-pyridin-2-ylméthyl-1 H-pyrazol-3-yl)-amide, l'acide 4-méthoxyméthyl-thiophène-2-carboxylique (5-méthyl-1-pyridin-2-ylméthyl-1H-pyrazol-3-yl)-amide, l'acide 3-chloro-4-méthanesulfonyl-thiophène-2-carboxylique thiazol-2-ylamide, l'acide 4-(4-chlorophényl)-thiophène-2-carboxylique thiazol-2-ylamide, l'acide 5-nitro-thiophène-3-carboxylique thiazol-2-ylamide, l'acide {2-[(5-propyl-thiophène-3-carbonyl)-amino]-thiazol-4-yl}-acétique, l'acide 5-propyl-thiophène-3-carboxylique (1H-benzoimidazol-2-yl)-amide, l'acide 4-(4-chlorobenzène-sulfonyl)-3-méthyl-thiophène-2-carboxylique thiazol-2-ylamide, l'acide 2-[(5-propyl-thiophène-3-carbonyl)-amino]-thiazole-4-carboxylique, l'acide 4-méthyl-thiophène-2-carboxylique (1-pyridin-2-ylméthyl-1 H-pyrazol-3-yl)-amide, l'acide 5-propyl-thiophène-3-carboxylique [1,3,4]thiadiazol-2-ylamide, l'acide 5-propyl-thiophène-3-carboxylique (1H-[1,2,4]triazol-3-yl)-amide, l'acide 5-méthyl-thiophène-2-carboxylique thiazol-2-ylamide, l'acide 4-méthyl-2-[(5-propyl-thiophène-3-carbonyl)-amino]-thiazole-5-carboxylique, l'acide 5-propyl-thiophène-3-carboxylique (5-fluoro-pyridin-2-yl)-amide, l'acide 5-propyl-thiophène-3-carboxylique (1-méthyl-1 H-pyrazol-3-yl)-amide, l'acide 5-amino-thiophène-3-carboxylique thiazol-2-ylamide, l'acide 5-propyl-thio-phène-3-carboxylique (5-tertio-butyl-2H-pyrazol-3-yl)-amide, l'acide 5-propyl-thiophène-3-carboxylique (4-chloro-pyridin-2-yl)-amide, l'acide 5-propyl-thiophène-3-carboxylique pyrimidin-2-ylamide, l'acide 4-éthyl-5-propyl-thiophène-2-carboxylique thiazol-2-ylamide, l'acide 5-propyl-thiophène-3-carboxylique (6-chloro-pyridazin-3-yl)-amide, l'acide 5-propyl-thiophène-3-carboxylique (2-éthyl-2H-pyrazol-3-yl)-amide, l'acide 5-propyl-thiophène-3-carboxylique pyrimidin-4-ylamide, l'acide 5-propyl-thiophène-3-carboxylique (1-pyridin-2-yl-méthyl-1 H-pyrazol-3-yl)-amide, l'acide 5-propyl-thiophène-3-carboxylique (1 H-pyrazol-3-yl)-amide, l'acide 5-propyl-thiophène-3-carboxylique (4-méthyl-pyridin-2-yl)-amide, l'acide {3-[(4-méthoxyméthyl-thiophène-2-carbonyl)-amino]-pyrazol-1-yl}-acétique éthylester, l'acide 5-propyl-thiophène-3-carboxylique pyridin-2-ylamide, l'acide 4-benzylsulfamoyl-5-méthyl-thiophène-2-carboxylique thiazol-2-ylamide, l'acide 4-diéthylsulfamoyl-5-méthyl-thiophène-2-carboxylique thiazol-2-ylamide, l'acide 5-méthyl-4-phénylsulfamoyl-thiophène-2-carboxylique thiazol-2-ylamide, l'acide 4-méthanesulfonyl-5-propoxy-thiophène-2-carboxylique thiazol-2-ylamide,
et/ou les sels, solvats et stéréoisomères de celui-ci, y compris des mélanges de ceux-ci en toutes proportions, et éventuellement des excipients et/ou des adjuvants.
